(19) Europäisches Patentamt
European Patent Office
Office européen des brevets

(11) EP 2 753 690 B1

(12) **FASCICULE DE BREVET EUROPEEN**

(45) Date de publication et mention de la délivrance du brevet:
**13.04.2016 Bulletin 2016/15**

(21) Numéro de dépôt: **12773126.3**

(22) Date de dépôt: **07.09.2012**

(51) Int Cl.:
***C12Q 1/00*** *(2006.01)* ***C12N 9/04*** *(2006.01)*

(86) Numéro de dépôt international:
**PCT/IB2012/054650**

(87) Numéro de publication internationale:
**WO 2013/035080 (14.03.2013 Gazette 2013/11)**

(54) **MUTANTS DE LA GLUCOSE OXYDASE DE PENICILLIUM AMAGASAKIENSE**

PENICILLIUM-AMAGASAKIENSE-GLUCOSEOXIDASE-MUTANTEN

PENICILLIUM AMAGASAKIENSE GLUCOSE OXIDASE MUTANTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.09.2011 FR 1157979**

(43) Date de publication de la demande:
**16.07.2014 Bulletin 2014/29**

(73) Titulaire: **Centre National de la Recherche Scientifique**
**75016 Paris (FR)**

(72) Inventeurs:
- **MANO, Nicolas**
**F-33400 Talence (FR)**
- **COURJEAN, Olivier**
**F-33320 Eysines (FR)**
- **TREMEY, Emilie**
**F-34150 Gignac (FR)**
- **GOUNEL, Sébastien**
**F-33124 Berthez (FR)**

(74) Mandataire: **Gevers & Orès**
**36 rue de Saint-Pétersbourg**
**75008 Paris (FR)**

(56) Documents cités:
**WO-A1-2011/068050**

- **SANDIP B BANKAR ET AL: "Glucose oxidase - An overview", BIOTECHNOLOGY ADVANCES, ELSEVIER PUBLISHING, BARKING, GB, vol. 27, no. 4, 1 juillet 2009 (2009-07-01), pages 489-501, XP002669514, ISSN: 0734-9750, DOI: 10.1016/J.BIOTECHADV.2009.04.003**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 753 690 B1

Printed by Jouve, 75001 PARIS (FR)

**Description**

La présente invention se rapporte au domaine de la mise au point d'électrodes à glucose qui présentent un intérêt dans le dosage du glucose, en particulier du glucose sanguin de sujets diabétiques, et pour la mise en oeuvre de biopiles utilisant le glucose comme combustible.

La présente invention vise plus particulièrement des mutants de l'enzyme glucose oxydase (aussi appelée GOx dans ce qui suit) de *Penicillium amagasakiense* qui présentent des propriétés avantageuses par rapport aux enzymes sauvages, en particulier celles commercialisées.

Le diabète de type 2 touche près de deux millions de personnes en France, auxquels s'ajoutent 600 000 qui ignorent leur maladie. Aux Etats-Unis, la situation est encore plus critique. Dans les pays développés, le diabète est la première cause de cécité chez les 20-65 ans.

Le suivi et la surveillance de la maladie repose, entre autres, sur le dosage quotidien du glucose sanguin et l'injection d'insuline. Différentes compagnies proposent des capteurs à glucose permettant aux patients de mesurer chez eux leur glycémie. Ces capteurs peuvent être ampérométriques, potentiométriques ou coulométriques ; ils reposent tous sur l'utilisation d'une enzyme capable d'oxyder le glucose ; les deux principales enzymes étant la glucose oxydase et la PQQ s-GDH.

La glucose oxydase (également dénommée GOx dans ce qui suit) est une enzyme oxydo-réductase (EC 1.1.3.4) qui catalyse l'oxydation du glucose en peroxyde d'hydrogène et en D-glucono-$\delta$-lactone selon le schéma réactionnel suivant :

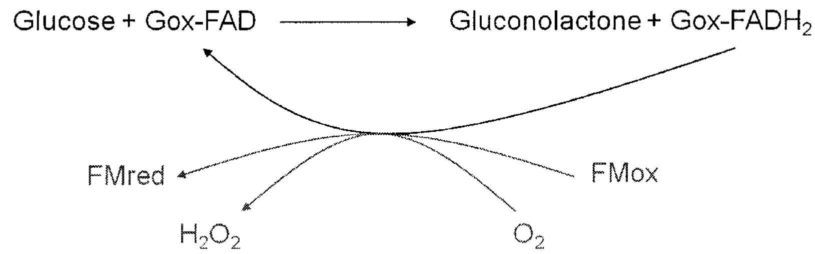


La glucose oxydase a été isolée pour la première fois chez *Aspergillus niger* ; les GOx les plus classiquement produites sont celles de *Penicillium chrysogenum, Penicillium glaucum, Penicillium purpurogenum, Penicillium amagasakiense, Aspergillus niger* et *Aspergillus fumaricus.*

Les GOx commercialisées sont le plus souvent celles d'*Aspergillus niger* et de *Penicillium amagasakiense* ; elles sont principalement utilisées dans l'industrie alimentaire, notamment à des fins de conservation comme source de peroxyde d'hydrogène. Elles sont également utilisées pour le dosage du glucose ou dans des biopiles à glucose. Ces deux enzymes ont notamment été étudiées et comparées dans l'article de Wohlfahart et al. (Acta. Cryst (1999) D55, 969-977).

S'agissant de la GOx de *Penicillium amagasakiense,* Witt *et al.* ont décrit son clonage et son expression par *Escherichia coli* (Applied and Environmental Microbiology (1998) vol. 64, n°4, 1405-1411).

L'inconvénient des glucose oxydases actuellement disponibles est leur sensibilité à l'$O_2$ qui intervient comme accepteur d'électrons dans la réaction catalysée par la GOx. En effet, l'oxygène est le co-facteur naturel des GOx et permet leur réoxydation après l'oxydation du glucose. Lors de l'utilisation de ces enzymes dans des capteurs à glucose, il existe donc une compétition pour récupérer les électrons de l'enzyme entre l'oxygène et les médiateurs redox qui assurent la connexion électrique de l'enzyme à la surface des électrodes.

En outre, pour leur utilisation dans des capteurs à glucose, il est nécessaire de disposer de mutants GOx plus actifs, c'est-à-dire qui permettent une réaction de transformation de glucose en D-gluconolactone plus rapide qu'avec les enzymes existantes.

Il demeure ainsi nécessaire d'améliorer les propriétés des GOx existantes.

C'est ce à quoi sont parvenus les Inventeurs en mettant au point de nouveaux mutants de la GOx sauvage de *Penicillium amagasakiense.*

Par mutant ou variant, on entend une GOx dont la séquence protéique comprend l'insertion, la délétion et/ou la substitution d'au moins un acide aminé par rapport à la séquence protéique de la GOx sauvage ; par la suite, les séquences nucléotidique et protéique de référence de la GOx sont celles de la GOx sauvage de *Penicillium amagasakiense* (respectivement SEQ. ID. N°1 et 2).

Les mutants selon la présente invention sont tels que la valine située en position 564 est substituée par une sérine (mutant dit V564S), une thréonine (mutant dit V564T) ou une isoleucine (mutant dit V564I) ; lorsque ladite valine est substituée par une sérine, les mutants tels que la lysine en position 424 est substitué par un acide glutamique, la glutamine, la méthionine et la leucine sont également des objets de la présente invention (mutants dits respectivement

V564S+K424E, V564S+K424Q, V564S+K424M et V564S+K424L).

Ainsi, un premier objet de l'invention se rapporte à un mutant GOx présentant un pourcentage d'identité d'au moins 95%, et par ordre de préférence croissant au moins 97%, 98% et 99%, par rapport à la GOx sauvage de *Penicillium amagasakiense,* caractérisé en ce que son acide aminé situé en position 564, en référence à la séquence protéique de la GOx sauvage de *Penicillium amagasakiense* (SEQ. ID. N°2), est substitué par un acide aminé sélectionné dans le groupe constitué par une sérine (mutant dit V564S), une thréonine (mutant dit V564T) ou une isoleucine (mutant dit V564I) et en ce que la sensibilité à l'oxygène dudit mutant est moindre que celle de la GOx sauvage de *Penicillium amagasakiense.*

Selon une variante particulière, le mutant V564S comporte également une substitution de la lysine en position 424 par un acide glutamique, la glutamine, la méthionine ou la leucine (mutants dits respectivement V564S+K424E, V564S+K424Q, V564S+K424M et V564S+K424L).

La numérotation des acides aminés se réfère à la séquence de la GOx sauvage de *Penicillium amagasakiense.*

L'identité d'une séquence par rapport à la séquence de la GOx sauvage de *Penicillium amagasakiense* (SEQ. ID. N°2) comme séquence de référence s'apprécie en fonction du pourcentage de résidus d'acides aminés qui sont identiques, lorsque les deux séquences sont alignées, de manière à obtenir le maximum de correspondance entre elles.

Le pourcentage d'identité peut être calculé par l'homme du métier en utilisant un programme informatique de comparaison de séquences tel que, par exemple celui de la suite BLAST (Altschul et al, NAR, 25, 3389-3402). Les programmes BLAST sont mis en oeuvre sur la fenêtre de comparaison constituée de la totalité de la SEQ. ID. N°2 indiquée comme séquence de référence.

Un peptide ayant une séquence en acides aminés ayant au moins X% d'identité avec une séquence de référence est défini, dans la présente invention comme un peptide dont la séquence peut inclure jusque 100-X altérations pour 100 acides aminés de la séquence de référence, tout en conservant les propriétés fonctionnelles dudit peptide de référence, en l'occurrence son activité enzymatique d'oxydation du glucose. Au sens de la présente invention, le terme altération inclut les délétions, les substitutions ou les insertions consécutives ou dispersées d'acides aminés dans la séquence de référence.

L'acide aminé correspondant à l'acide aminé situé en position 564 de la GOx sauvage de *Penicillium amagasakiense* est identifié par alignement de la séquence de ladite enzyme homologue avec la GOx de *Penicillium amagasakiense.*

Un objet particulier de l'invention se rapporte à un mutant GOx ayant une séquence en acides aminés choisie parmi les SEQ. ID. N° 4, 6, 8, 10, 22, 24 et 26, correspondants respectivement aux séquences en acides aminés des mutants V564S, V564T, V564I, V564S+K424E, V564S+K424Q, V564S+K424M et V564S+K424L de la GOx ; ces enzymes mutées sont codées par des fragments nucléotidiques obtenus par mutation du gène de la GOx sauvage de *Penicillium amagasakiense* avec des paires d'oligonucléotides adaptées.

Ces nouveaux mutants GOx selon l'invention présentent des performances améliorées par rapport à l'enzyme sauvage d'*Aspergillus niger* qui est l'enzyme utilisée dans les capteurs de glucose du commerce.

Plus particulièrement, les propriétés améliorées des mutants selon l'invention résident dans une moindre sensibilité à l'oxygène : en solution en présence de 1 mM de glucose et sous air, les mutants sont 17 fois moins sensibles à l'oxygène que la GOx d'A. *niger.* Une fois adsorbés à la surface d'électrodes, sous 1 atm $O_2$ et à 1 mM de glucose, les mutants sont 70% moins sensibles à l'$O_2$.

Les propriétés avantageuses des mutants GOx selon l'invention rendent leur utilisation particulièrement adaptée à des systèmes bioélectriques tels que des biopiles utilisant le glucose comme source d'énergie et des biocapteurs de glucose.

La présente invention se rapporte également à une molécule d'acide nucléique codant pour un mutant GOx selon l'invention ; ladite molécule d'acide nucléique étant obtenue par modification d'une GOx sauvage, telle que celle de *Penicillium amagasakiense,* avec une paire d'oligonucléotides sélectionnée dans le groupe constitué des paires d'oligonucléotides représentées dans le Tableau I.

Tableau I: liste et séquence des oligonucléotides utilisés pour la préparation des mutants GOx selon l'invention.

| Oligonucléotides | séquences |
|---|---|
| Paire d'oligonucléotides correspondant à l'enzyme sauvage | |
| Sens | 5'-g gtg tct tcc cat **gtc** atg acc att ttc tac gg-3' (SEQ. ID. N°11) |
| Anti-sens | 5'- cc gta gaa aat ggt cat **gac** atg gga aga cac c-3' (SEQ. ID. N°12) |
| Paire d'oligonucléotides utilisés pour la préparation du mutant V564S | |

(suite)

| Oligonucléotides | séquences |
|---|---|
| Sens | 5'- g gtg tct tcc cat **tcc** atg acc att ttc tac gg-3' (SEQ. ID. N°13) |
| Anti-sens | 5'- cc gta gaa aat ggt cat **gga** atg gga aga cac c-3' (SEQ. ID. N° 14) |
| Paire d'oligonucléotides utilisés pour la préparation du mutant V564T | |
| Sens | 5'- g gtg tct tcc cat **acc** atg acc att ttc tac gg-3' (SEQ. ID. N°15) |
| Anti-sens | 5'- cc gta gaa aat ggt cat **ggt** atg gga aga cac c-3' (SEQ. ID. N°16) |
| Paire d'oligonucléotides utilisés pour la préparation du mutant V564I | |
| Sens | 5'- g gtg tct tcc cat **att** atg acc att ttc tac gg-3' (SEQ. ID. N°17) |
| Anti-sens | 5'- cc gta gaa aat ggt cat **aat** atg gga aga cac c-3' (SEQ. ID. N°18) |
| Paire d'oligonucléotides utilisés pour la préparation de la mutation K424E du mutant V564S-K424E (cette mutation est réalisée après la mutation V564S) | |
| Sens | 5'- ggacaccgagggcgagatcaacttcg-3' (SEQ. ID. N°19) |
| Anti-sens | 5'- cgaagttgatctcgccctcggtgtcc-3' (SEQ. ID. N°20) |
| Paire d'oligonucléotides utilisés pour la préparation de la mutation K424Q du mutant V564S-K424Q (cette mutation est réalisée après la mutation V564S) | |
| Sens | 5'-ggacaccgagggccagatcaacttcgatttatg-3' (SEQ. ID. N°27) |
| Anti-sens | 5'-cataaatcgaagttgatctggccctcggtgtcc-3' (SEQ. ID. N°28) |
| Paire d'oligonucléotides utilisés pour la préparation de la mutation K424M du mutant V564S-K424M (cette mutation est réalisée après la mutation V564S) | |
| Sens | 5'-ggacaccgagggcatgatcaacttcgatttatg-3' (SEQ. ID. N°29) |
| Anti-sens | 5'-cataaatcgaagttgatcatgccctcggtgtcc-3' (SEQ. ID. N°30) |
| Paire d'oligonucléotides utilisés pour la préparation de la mutation K424L du mutant V564S-K424L (cette mutation est réalisée après la mutation V564S) | |
| Sens | 5'-ggacaccgagggcttgatcaacttcgatttatg-3' (SEQ. ID. N°31) |
| Anti-sens | 5'-cataaatcgaagttgatcaagccctcggtgtcc-3' (SEQ. ID. N°32) |

Les molécules d'acide nucléique codant pour les mutants GOx selon l'invention peuvent être notamment préparées en modifiant la séquence nucléotidique du gène codant pour l'enzyme sauvage de séquence SEQ. ID. N°1 produite par *Penicillium amagasakiense.* Plusieurs techniques permettant la modification de la séquence du gène sont connues de l'homme du métier (voir la revue de Igarashi et al., Archives of Biochemistry and Biophysocs 428 (2004) 52-63). Selon un mode particulier de préparation, les molécules d'acide nucléique codant pour les mutants GOx selon l'invention sont préparées par mutagénèse par PCR en présence d'un oligonucléotide portant la mutation à introduire (voir la partie expérimentale, point 2.5 ci-après).

Selon un mode de réalisation particulier, la présente invention se rapporte à une molécule d'acide nucléique codant pour un mutant GOx selon l'invention dont la séquence est sélectionnée dans le groupe constitué des séquences SEQ. ID. N°3, 5, 7, 9, 21, 23 et 25. Les molécules d'acide nucléique codant pour les mutants GOx selon l'invention peuvent alors être clonées dans un vecteur d'expression tel qu'un plasmide, puis transformé dans un hôte approprié tel qu'une bactérie, une levure ou encore une culture cellulaire.

Par vecteur d'expression, on entend un vecteur possédant une région permettant l'insertion d'une séquence nucléotidique codante entre les signaux indispensables à son expression, notamment, un promoteur (constitutif ou

inductible), un site de fixation des ribosomes, un signal de terminaison de transcription et, éventuellement, un marqueur de sélection tel qu'un gène de résistance à un antibiotique.

La présente invention se rapporte encore à un vecteur d'expression comprenant ladite molécule d'acide nucléique et à une cellule hôte transformée avec ledit vecteur d'expression et exprimant un mutant GOx selon l'invention.

L'introduction du vecteur d'expression dans la cellule hôte peut être réalisée par toute méthode connue de l'homme du métier, en particulier, par modification de la perméabilité membranaire de la cellule hôte, par exemple en présence d'ions calcium, ou par électroporation.

Après culture des cellules hôtes transformées pour exprimer un mutant GOx selon l'invention, lesdites cellules peuvent être récupérées par centrifugation, lysées afin de libérer les enzymes dont ledit mutant GOx selon l'invention.

Si *Escherichia coli* est le micro-organisme hôte, les plasmides qui peuvent être utilisés sont notamment les plasmides pET24a, pBluescript, pUC18 ou similaires.

A titre d'exemple, les cellules hôtes qui peuvent être utilisées comprennent d'*Escherichia coli* $BL_{21}$, *Escherichia coli* W3110, *Escherichia coli* C600, *Escherichia coli* JM109, *Escherichia coli* JM101, *Escherichia coli* DH5$\alpha$...

De préférence, les mutants GOx selon l'invention sont produits dans une souche d'*Escherichia coli* $BL_{21}$ ; la molécule d'acide nucléique qui les code est obtenue par modification du gène de la GOx de *Penicillium amagasakiense* et clonée dans le vecteur pET24a. Les mutants ainsi produits sont exportés dans le périplasme de la bactérie grâce à la séquence signal de la GOx. Les mutants ainsi produits sont exportés dans les corps d inclusion de la bactérie. Les mutants sont ensuite purifiés après rupture des bactéries par lyse cellulaire en présence d'urée 8M.

L'invention se rapporte également à l'utilisation d'un mutant GOx selon l'invention pour doser le glucose en solution, c'est-à-dire mesurer la concentration en glucose dans un échantillon, notamment un échantillon biologique, en particulier, dans du sang.

Le dosage en solution du glucose dans un échantillon biologique donné peut être réalisé en introduisant dans ledit échantillon un réactif d'oxydoréduction et un mutant GOx selon l'invention puis en comparant l'intensité de la coloration obtenue avec des solutions standards ayant une teneur en glucose connue.

La présente invention se rapporte également à un kit de dosage en solution du glucose caractérisé en ce qu'il comprend un mutant GOx selon l'invention.

Typiquement, ledit kit de dosage contient en outre les réactifs nécessaires à la mise en oeuvre du test de dosage du glucose, en particulier, les tampons ; tout tampon peut être utilisé dans le kit selon l'invention, on peut citer sans caractère limitatif les tampons phosphates, actétates, tampon au trishydroxyméthylaminométhane (TRIS), à l'acide N-morpholino-3-propane sulfonique (MPOS), à l'acide 4-(2-hydroxyéthyl)-1-pipérazine éthane sulfonique (HEPES), tampon comprenant un mélange de tampons tel que TRIS-acétate..., les réactifs d'oxydoréduction peuvent être tout réactif permettant d'oxyder le mutant GOx, ils peuvent être sélectionnés dans le groupe constitué par du méthosulfate de phénazine (PMS) en combinaison avec le 2,6-dichlorophénolindophénol (DCIP) ; du ferricyanure de potassium ; du ferrocène et des complexes dérivés du ferrocène tels que ferrocèneméthanol, ferrocène carboxylique acide ; et des complexes d'osmium et de ruthénium, les solutions standard de glucose permettant la réalisation de courbes d'étalonnage, et les instructions d'utilisation nécessaires pour effectuer le dosage.

La présente invention se rapporte encore à des électrodes à glucose comprenant un matériau conducteur tel qu'un métal conducteur, notamment, du platine, du cuivre, de l'argent, de l'aluminium, de l'or ou de l'acier ou du carbone, comme du carbone vitreux, des fibres de carbone, des fibres de nanotubes de carbone ou encore en diamant... ledit matériau conducteur est recouvert d'un dépôt comprenant au moins un mutant GOx selon l'invention ; ledit dépôt pouvant en outre comprendre un polymère redox pour améliorer les propriétés conductrices du matériau conducteur.

Le polymère redox est choisi parmi les polymères à base de ferrocène, d'osmium et de ruthénium et les polymères conducteurs tels que le polypyrrole et la polyananilline.

Les méthodes d'immobilisation du mutant GOx sur ledit matériau conducteur peuvent être choisies parmi les méthodes classiques à la disposition de l'homme du métier qui comprennent notamment l'inclusion du mutant GOx dans une matrice polymérique, l'adsorption du mutant GOx à la surface de la membrane polymérique, la fixation par liaison covalente ou encore l'électrodéposition (Gao et al., Chem. Int. ED. 2002, 41, N°5, 810-813).

De telles électrodes sont avantageusement utilisées dans des systèmes bioélectriques tels que des biopiles à glucose ou des biocapteurs de glucose.

La présente invention se rapporte ainsi également à un biocapteur de glucose comprenant une électrode selon l'invention.

Un biocapteur de glucose est constitué d'une électrode sur laquelle est immobilisé un biorécepteur capable de reconnaître une cible biologique ; la fixation de la cible biologique sur le biorécepteur conduit à des modifications physico-chimiques de la membrane et la production d'un signal électrique par un transducteur électrochimique (ampérométrique, potentiométrique, conductimétrique,...) accolé à l'électrode ; dans le cas présent le biorécepteur est un mutant GOx selon l'invention et la cible biologique est son substrat : le glucose.

Selon une variante de réalisation, l'électrode sur laquelle est immobilisée le mutant GOx est également recouverte d'une membrane qui évite le détachement dudit mutant de l'électrode. Ladite membrane peut être constituée de

nafion, de cellulose ou de tout matériau biocompatible, c'est-à-dire compatible avec un environnement physiologique.

Selon une variante de l'invention, le biocapteur de glucose est implanté sous la peau et permet d'enregistrer la concentration en glucose du sang.

La présente invention se rapporte également à des biopiles utilisant du glucose comme source d'énergie et comprenant une première électrode selon l'invention à titre d'anode et une seconde électrode à titre de cathode. La cathode peut être, par exemple, une électrode enzymatique qui permet de réduire l'oxygène portant une enzyme choisie dans la classe des enzymes à base de cuivre (multi coppers oxydases) et particulièrement la bilirubine oxydase et la laccase. Il peut également s'agir d'une électrode métallique, par exemple en platine, en or ou en un alliage de platine ou d'or.

La **Figure 1** illustre plus spécifiquement une biopile enzymatique à glucose ; une telle biopile enzymatique consiste en deux électrodes modifiées par l'immobilisation d'enzymes. Une glucose oxydase (GOx) est fixée sur l'anode (1) par l'intermédiaire d'un polymère conducteur « *I* » et une bilirubine oxydase (BOD) est fixée sur la cathode (2) par l'intermédiaire d'un polymère conducteur *« II »*. En fonctionnement, à l'anode, les électrons sont transférés du glucose présent dans le fluide physiologique vers la GOx, puis de la GOx au polymère conducteur « *I »* et du polymère conducteur « *I »* vers l'anode. A la cathode, les électrons sont transférés de la cathode vers le polymère conducteur *« II »,* puis vers la BOD et enfin de la BOD à l'oxygène présent dans le fluide physiologique.

Il est à noter qu'une biopile peut aussi éventuellement fonctionner en modifiant les électrodes avec leurs enzymes respectives et en rajoutant des médiateurs solubles, tels que le ferrocèneméthanol pour l'anode et le ferricyanure de potassium pour la cathode, et en rajoutant le cas échéant une membrane séparant l'anode et la cathode.

L'invention se rapporte encore à un procédé de dosage en solution du glucose dans un échantillon comprenant les étapes suivantes :

a) introduction dans ledit échantillon d'un réactif d'oxydoréduction dont la réduction conduit à un changement de couleur et d'un mutant GOx selon l'invention ;
b) mesure de l'intensité de la coloration de l'échantillon après réaction enzymatique ;
c) comparaison de l'intensité de coloration mesurée à l'étape b) avec l'intensité mesurée pour des solutions standards ayant une teneur en glucose connue ;
d) détermination de la concentration en glucose dudit échantillon.

Le réactif d'oxydoréduction dont la réduction conduit à un changement de couleur est choisi parmi le phénazine méthosulfate (PMS) en combinaison avec le 2,6-dichlorophénolindophénol (DCIP), le ferricyanure de potassium, et le ferrocène.

L'invention se rapporte également à un procédé de dosage du glucose d'un échantillon, caractérisé en ce qu'il comprend les étapes suivantes :

a) introduction dans ledit échantillon d'une électrode à glucose selon l'invention ;
b) mesure de l'intensité du courant dans l'échantillon ;
c) comparaison de l'intensité du courant mesurée à l'étape b) avec l'intensité mesurée pour des solutions standards ayant une teneur en glucose connue ;
d) détermination de la concentration en glucose dudit échantillon.

Outre les dispositions qui précèdent, l'invention comprend encore d'autres dispositions qui ressortiront de la description qui va suivre, qui se réfèrent à des exemples de mise en oeuvre de la présente invention, ainsi qu'aux figures annexées dans lesquelles :

<u>**Figures**</u>

La **Figure 1** représente schématiquement une biopile.

La **Figure 2** représente la carte plasmidique du vecteur pET24a-GOx-penag-wt-His.

La **Figure 3** est un graphe illustrant l'activité spécifique en U/mg des GOx sauvage et mutantes *de Penicillium amagasakiense.*

La **Figure 4** est un graphe représentant le rapport Activité Ferrocèneméthanol / Activité Oxygène.

La **Figure 5** montre une comparaison de l'activité spécifique des GOx sauvage et mutantes avec le glucose et le xylose.

La **Figure 6** est un graphe représentant l'évolution du courant d'oxydation du glucose en fonction de la concentration de glucose.

La **Figure 7** est un graphe représentant l'évolution du ratio courant d'oxydation du glucose sous oxygène divisé par le courant sous Argon en fonction de la concentration en glucose.

## 1. Matériels

### *1.1 Souches bactériennes d'Escherichia coli*

**$DH_5\alpha$ :** *sup*E44, Δ*lac*U169, (Φ80 *lac*ZDM15), *hsd*R17, *rec*A1, *end*A1, *gyr*A96, *thi*-1, *rel*A1 (Hanahan, 1983). Cette souche est utilisée pour l'amplification de plasmide lors des étapes de construction des vecteurs d'expression protéiques.

**$BL_{21}$** : F- *ompT hsd*SB(rB-, mB-) *gal dcm* (DE3) (Invitrogen). Cette souche est utilisée pour la production en flacon Erlenmeyers de la GOx de *Penicillium Amagasakiense (penag).* Cette souche est alors transformée par le plasmide pET24a qui contient la séquence d'ADN codant pour la GOx de *Penicilium Amagasakiense* sous la dépendance du promoteur T7 dans le vecteur pET24a.

### *1.2 Vecteur*

**pET24a :** Plasmide pET24a contenant la séquence ADN codant pour la GOx de *Penicilium Amagasakiense* clonée en phase avec l'étiquette 6xHis en C-terminal (la carte de ce plasmide est représentée à la **Figure 2**).

### *1.3 Milieu de culture*

Milieu riche LB :
Tryptone 10 g/l
Extrait de levure 5 g/l
NaCl g/l
$H_2O$ distillée qsp 1L
pH non ajusté, autoclavé pendant 50 min à 1 bar.

## 2. Techniques de génie génétique

### *2.1 Préparation des bactéries électrocompétentes*

5 ml de préculture de cellule DH5α sont inoculées dans 1 L de LB et sont mises en culture à 37°C jusqu'en phase exponentielle (DO entre 0,6 et 0,8). Les cellules ainsi récoltées par centrifugation à 4000 g sont lavées successivement avec de l'eau froide milliQ® jusqu'à obtenir 2 ml de cellule devenues électrocompétentes.

### *2.2 Transformation des bactéries électrocompétentes*

1 μl d'ADN plasmidique est incorporé dans 40 μl de cellules électrocompétentes, placé dans une cuve d'électroporation et immédiatement transformé par l'électroporateur. 500 μl de SOC (milieu de culture contenant 2 % tryptone, 0,5 % yeast extract, 10 mM NaCl, 2,5 mM KCl, 10 mM $MgCl_2$, 10 mM MgSO4) sont rajoutés, incubés 5 minutes dans la glace et mis en culture lh à 37°C. Les 500 μL de cultures sont ensuite déposés sur boite LB-agar et incubés sur la nuit à 37°C.

### *2.3 Préparation de l'ADN*

Cette étape est réalisée à l'aide du kit QIAprep® Miniprep (Qiagen) qui permet d'extraire et de purifier l'ADN plasmidique à partir de 10 ml de culture de cellules DH5α transformées avec le plasmide désiré. Après la collecte des cellules par centrifugation, celles-ci subissent une lyse alcaline, en présence de RNase, ainsi qu'une précipitation de l'ADN génomique par l'acide acétique. L'ADN est alors éliminé par centrifugation et le surnageant déposé sur une colonne comportant une matrice de silice, permettant une adsorption sélective de l'ADN plasmidique en présence d'une forte concentration en sel. Après un lavage à l'éthanol pour éliminer les sels, l'ARN et les protéines, l'ADN plasmidique est élué avec un tampon de faible force ionique (eau ou tampon: Tris-HCl 10 mM pH 8,5). L'ADN ainsi purifié peut être quantifié par spectrométrie UV-visible à 260 nm. Une absorbance de 1 correspond à une concentration d'ADN de 50 ng.$\mu l^{-1}$. L'ADN plasmidique purifié est stocké à -20°C.

### *2.4 Digestion de l'ADN*

Pour une digestion totale, 200 à 500 ng d'ADN plasmidique sont digérés avec 0,5 µl de l'enzyme de restriction XbaI dans le tampon de réaction approprié, dans un volume final de 15 µl. La réaction se fait à 37°C pendant 1 heure.

### *2.5 Mutagenèse dirigée par PCR*

Les mutants de la GOx V564I, V564S, V564T et V564S+K424E sont obtenus par mutagénèse dirigée. Cette méthode nécessite l'utilisation d'un plasmide double brin (le plasmide pET24a) portant le gène d'intérêt (*GOx*) ainsi que 2 oligonucléotides synthétiques dont la séquence est complémentaire au brin d'ADN à modifier, à l'exception de la mutation désirée. Ces oligonucléotides contiennent entre 25 et 45 bases, avec une température de fusion (Tm) supérieure ou égale à 70°C.

$$Tm = 81,5 + 0,41\ (\%\ GC) - 675/N - \%\ (\text{non appariement})$$

Avec N le nombre de bases de la séquence, % GC le pourcentage de bases G et C dans la séquence et % (non appariement) le nombre de bases mutées (valeur nulle en cas de délétion ou d'insertion de base). La séquence choisie doit contenir au moins 40 % de bases GC et se terminer par un C ou un G.

Les séquences des oligonucléotides utilisés sont présentées dans le Tableau I ci-avant.

Dans un flacon Eppendorf stérile, sont mélangés 10 ng de plasmide parental, 12,5 ng de chacune des amorces, 1 µl de mélange de dNTP concentrés à 10 mM, 5 µl de tampon de réaction, 1µl de ADN polymérase *Pfu Turbo* (2,5 $U.\mu l^{-1}$) et 50 µl q.s.p d'eau stérile.

La mutagénèse s'effectue par un enchaînement de cycles de température réalisés de manière automatique par un thermocycleur. Chaque cycle comporte 3 étapes. Dans un premier temps les 2 brins de l'ADN matrice sont séparés par dénaturation thermique, puis les oligonucléotides s'apparient à leur séquence complémentaire sur l'ADN matrice. Ils servent d'amorces pour l'étape d'élongation, au cours de laquelle la *PfuTurbo* polymerase (une ADN polymérase thermorésistante) synthétise l'ADN complémentaire au brin parental.

Une fois cet enchaînement de cycles terminé, le produit de la réaction est traité par la *Dpn I,* une endonucléase qui digère spécifiquement l'ADN méthylé et hémiméthylé du plasmide parental. L'ADN muté est finalement introduit dans des cellules compétentes qui relient les extrémités du plasmide encore libres après la synthèse de l'ADN.

### *2.6 Séquençage de l'ADN double brin*

L'ADN double brin est séquencé à la plateforme génomique de l'université Victor Segalen. Les réactions de séquences sont effectuées avec le kit de séquençage BigDye Terminator v1.1 ou v3.1. Le réactif contient les 4 ddNTPs avec différents marqueurs fluorescents (BigDyeTerminators), l'AmpliTaq DNA Polymerase, et tous les autres composants nécessaires à la réaction. Les produits d'extension doivent être purifiés avant le passage sur séquenceur ABI 3130x1, pour éliminer les marqueurs non incorporés, sels et autres contaminants.

## 3. Production et purification de l'enzyme Glucose oxydase de *Penicillium amagasakiense*

### *3.1 Production des enzymes GOx de type sauvage et mutées*

L'enzyme GOx est produite dans la souche *E. coli* BL21 par le plasmide recombinant pET24a portant la séquence codant la GOx sauvage ou mutée. Une préculture de 2 ml de milieu LB supplémenté en kanamycine (1 X) est ensemencée par un clone isolé sur boîte LB agar supplémenté en kanamycine (1 X) et laissée sous agitation, à 220 rpm, sur la nuit à 37°C. Une culture de 50 ml est alors ensemencée au $1/25^{\text{ème}}$ en milieu LB supplémenté en kanamycine (1X) dans un Erlenmeyer de 250 ml. Celui-ci est incubée à 37°C sous agitation (220 rpm) jusqu'à une $DO_{600nm}$ comprise entre 0.8 et 1 $DO_{600}$nm /ml. La culture est ensuite induite par 500 µM d'IPTG puis laissée sous agitation (220 rpm) à 37°C pendant 2 heures.

### *3.2 Préparation des extrait solubles*

Les cellules récoltées par centrifugation (4500g, 4°C) sont tout d'abord lavées dans 5 ml de tampon Tris/HCl 20 mM; NaCl 100 mM ; EDTA 1 mM. Les cellules récoltées par centrifugation (4500g, 4°C) sont ensuite lavées dans 5 ml de tampon Tris/HCl 20 mM; NaCl 100 mM ; EDTA 1 mM contenant de l'urée à 3M afin de fragiliser la membrane des

cellules. Les cellules récoltées (4500g, 4°C) sont incubées 1 heure sur la glace en présence de 5 ml de tampon Tris/HCl 20 mM; NaCl 100 mM ; EDTA 1 mM contenant de l'urée 8M permettant la lyse complète des cellules. Le surnageant est récolté après centrifugation à 4500g à 4°C puis stockées à -20°C.

### *3.3 Reconstitution de la GOx*

Dans la bactérie *E. coli* BL21, la Glucose oxydase de *Penicillium amagasakiense* est surexprimée sous sa forme Apo, c'est-à-dire en absence de son cofacteur la Flavine Adénine Dinucléotide (FAD). Il est donc nécessaire de la reconstituer chimiquement. Pour cela les 5 ml d'extrait soluble obtenu sont ajoutés goutte à goutte sous agitation forte, afin d'éviter la précipitation des protéines, dans 500 ml d'une solution de reconstitution contenant 10% de glycérol, 1 mM de glutathion réduit, 1 mM de glutathion oxydé, 100 μM de FAD dans du tampon Tris/HCl 20 mM pH 8. Cette solution est stockée pendant 5 jours à 4°C à l'abri de la lumière.

### *3.4 Purification de la GOx*

Chromatographie échangeuse d'anions

La solution de reconstitution, dialysée dans du tampon Acétate 20 mM pH 6 pour permettre la précipitation de la forme Apo restante, et concentrée jusqu'à 5 ml sur membrane Amicon YM10 puis filtré sur un filtre de 0,22 μm. Cette solution est injectée sur une colonne échangeuse d'anion QFF (GE Healthcare®), couplée au système AKTA purifier (GE Healthcare®), équilibrée dans un tampon sodium acétate 20 mM pH 6. L'élution est réalisée par un gradient de 0 % à 30 % d'un tampon sodium acétate 50 mM, NaCl 250 mM, pH 3 sous un débit de 1 ml/min. Les fractions contenant la protéine GOx sont identifiées par un test d'activité à l'ABTS et sont rassemblées, concentrées et dessalées avec un tampon phosphate 100 mM pH5 par centrifugation sur membrane Amicon YM10. A ce stade, la protéine GOx est pure et peut être conservée à 4°C sous forme soluble.

## 4. Caractérisation des enzymes GOx de type sauvage et mutées

### *4.1 Mesure de la concentration*

La détermination de la concentration des protéines se fait par spectroscopie UV-Visible à l'aide d'un spectrophotomètre Varian dans un tampon phosphate 100 mM pH 5 à 25°C. Les protéines GOx purifiées présentent un spectre caractéristique entre 200 et 800 nm. La première bande à 280 nm est caractéristique de l'absorption des acides aminés aromatiques et permet d'obtenir la concentration en enzyme ($\varepsilon = 263\ mM^{-1}.cm^{-1}$). Les deux autres bandes à 380 et 461 nm sont caractéristiques du FAD intégré dans la protéine. La valeur de l'absorbance à 461 nm, permet d'obtenir la concentration en cofacteur présent dans la protéine ($\varepsilon = 12,83\ mM^{-1}.cm^{-1}$). Le rapport entre la concentration en GOx et la concentration en FAD est égal à 2 lorsque la protéine est complètement reconstituée.

### *4.2 Test enzymatique*

Les tests enzymatiques sont effectués par spectroscopie UV-Visible à l'aide d'un spectrophotomètre Varian sous air.

Les tests enzymatiques sont réalisés en présence d'un fort excès de glucose (150 mM) afin de pouvoir observer uniquement la réoxydation de la GOx par les médiateurs.

Deux tests enzymatiques sont réalisés. Le premier avec un mélange ABTS-HRP (Acide 2,2'-azino-bis(3-ethylbenzthiazoline-6-sulphonique) - Horse radish peroxydase) pour pouvoir observer la réoxydation par l'oxygène. L'ABTS est oxydé en présence d'HRP et d'$H_2O_2$. Et le second avec du Ferrocèneméthanol oxydé, afin de pouvoir observer la réoxydation de la GOx par un médiateur redox ayant un potentiel redox proche de celui utiliser en électrochimie.

#### 4.2.1 Test enzymatique ABTS-HRP

Les tests sont réalisés dans un tampon phosphate 100 mM pH 5 à 37°C dans un volume de 3 ml contenant 100 μl de HRP (60 U/ml), 24 μL d'ABTS (11,5 mg/ml) et 150 mM glucose. L'oxydation de l'ABTS est suivi à 405 nm en fonction du temps ($s = 36,8\ mM^{-1}.cm^{-1}$). L'activité spécifique de l'enzyme est exprimée en micromole de produit apparu par mg d'enzyme (U/mg). L'enzyme est diluée à 50 nM de façon à mesurer une pente comprise entre 0,05 et 0,3 DO $_{405nm}$/min.

4.2.2 Test enzymatique Ferrocèneméthanol (FMox)

**4.2.2.1 Préparation du ferrocèneméthanol**

Dans le commerce le ferrocèneméthanol est sous forme réduite, il est donc nécessaire de l'oxyder pour pouvoir l'utiliser dans les tests enzymatiques. Pour cela 100 mg de ferrocèneméthanol réduit sont dissous dans 50 ml de tampon phosphate 50 mM pH 7,5 et placé dans une cellule d'électrolyse comprenant une électrode de travail (électrode de carbone), une électrode de référence Ag/AgCl, une contre-électrode de platine placée dans un fritté contenant du tampon phosphate 50 mM pH 7,5. Le système est sous argon. L'électrolyse se fait à 0,5 V pendant 4 heures.

**4.2.2.2 Test enzymatique**

Les tests sont réalisés dans un tampon phosphate 50 mM pH 7,5 à 37°C dans un volume de 3 ml contenant 1 mM de FMox et 150 mM glucose. La réduction du ferrocèméthanol est suivi à 625 nm en fonction du temps (s = 0.413 $mM^{-1}.cm^{-1}$). L'activité spécifique de l'enzyme est exprimée en micromole de produit apparu par mg d'enzyme (U/mg) (**Figure 3**). L'enzyme est diluée à 50 nM de façon à mesurer une pente comprise entre 0,001 et 0,1 $DO_{625nm}$/min.

On note que les quatre mutants montrent une nette diminution de l'activité vis-à-vis de l'oxygène par rapport à la GOx sauvage.

L'activité vis à vis du ferrocèneméthanol est différente selon les mutants. Ainsi le mutant V564S présente une activité plus faible, le mutant V564I présente une activité similaire, alors que le mutant V564T montre une forte augmentation de l'activité par rapport à la GOx sauvage.

Le rapport Activité FM ox / Activité ABTS permet d'obtenir la spécificité de l'enzyme vis-à-vis du substrat (ABTS ou FM ox). Plus le rapport est grand, moins l'enzyme est sensible à l'oxygène (**Figure 4**).

On remarque que les mutants V564S et V564T sont très peu sensibles à l'oxygène, ce qui en fait des bons candidats pour leur utilisation dans les systèmes électrochimiques.

Les mêmes tests enzymatiques ont été réalisés en présence de xylose (**Figure 5**) : les activités ABTS et FM ox pour le xylose sont très faibles par rapport aux activités pour le glucose. Le xylose ne rentre pas en compétition avec le glucose comme substrat pour les Glucose oxydases de *Penicillium amagasakiense* sauvage et mutantes.

**5. Tests électrochimiques**

A titre d'exemple, la **Figure 6** représente l'évolution du courant d'oxydation du glucose en fonction de la concentration de glucose dans un tampon PBS sous Argon et à 37°C. Chaque électrode est composée de 5% en masse de glucose oxydase, 10% en masse de réticulant et 75 % en masse de polymère rédox. La **Figure 7** représente l'évolution du ratio courant d'oxydation du glucose sous oxygène divisé par le courant sous Argon en fonction de la concentration en glucose. Cette figure montre bien une diminution de l'effet de l'oxygène sur l'oxydation du glucose lorsqu'on utilise les enzymes mutées.

## Revendications

1. Mutant de la Glucose Oxydase (GOx) présentant un pourcentage d'identité d'au moins 95%, par rapport à la GOx sauvage de *Penicillium amagasakiense,* **caractérisé en ce que** son acide aminé situé en position 564, en référence à la séquence protéique de la GOx sauvage de *Penicillium amagasakiense* de SEQ. ID. N°2, est substitué par un acide aminé sélectionné dans le groupe constitué par une sérine (mutant dit V564S), une thréonine (mutant dit V564T) ou une isoleucine (mutant dit V564I) et **en ce que** la sensibilité à l'oxygène dudit mutant est moindre que celle de la GOx sauvage de *Penicillium amagasakiense.*

2. Mutant de la GOx selon la revendication 1, **caractérisé en ce que** le mutant V564S comporte également une substitution de la lysine en position 424 par un acide glutamique (mutant dit V564S+K424E), la glutamine (mutant dit V564S+K424Q), la méthionine (mutant dit V564S+K424M) ou la leucine (mutant dit V564S+K424L).

3. Mutant de la GOx selon la revendication 1 ou la revendication 2, **caractérisée en ce qu'**il a une séquence en acides aminés sélectionnée dans le groupe constitué par les SEQ. ID. N°4, 6, 8, 10, 22, 24 et 26.

4. Molécule d'acide nucléique, **caractérisée en ce qu'**elle code pour un mutant GOx selon l'une quelconque des revendications 1 à 3.

5. Molécule d'acide nucléique selon la revendication 5, **caractérisée en ce qu'**elle est obtenue par mutation de la molécule d'acide nucléique de séquence SEQ. ID. N°1 avec une paire d'oligonucléotides sélectionnée dans le groupe constitué par les paires de SEQ. ID. N° 13 et 14; 15 et 16 ; 17 et 18; 19 et 20 ; 27 et 28 ; 29 et 30 et 31 et 32.

6. Molécule d'acide nucléique selon la revendication 4 ou la revendication 5, **caractérisée en ce qu'**elle a une séquence sélectionnée dans le groupe constitué par les SEQ. ID. N°3, 5, 7, 9, 21, 23 et 25.

7. Vecteur d'expression, **caractérisé en ce qu'**il comprend une molécule d'acide nucléique selon l'une quelconque des revendications 4 à 5.

8. Cellule hôte exprimant une enzyme selon l'une quelconque des revendications 1 à 3, **caractérisée en ce qu'**elle est transformée avec un vecteur d'expression selon la revendication 7.

9. Utilisation d'un mutant GOx selon l'une quelconque des revendications 1 à 3 pour mesurer la concentration en glucose dans un échantillon.

**10.** Utilisation selon la revendication 9, **caractérisée en ce que** l'échantillon est biologique, en particulier, est du sang.

**11.** Kit de dosage du glucose **caractérisé en ce qu'**il comprend un mutant de la GOx selon l'une quelconque des revendications 1 à 3.

**12.** Electrode à glucose, **caractérisée en ce qu'**elle comprend un matériau conducteur recouvert d'un dépôt comprenant au moins un mutant de la GOx selon l'une quelconque des revendications 1 à 3.

**13.** Capteur de glucose, **caractérisé en ce qu'**il est constitué d'une électrode selon la revendication 12.

**14.** Biopile à glucose, **caractérisée en ce qu'**elle comprend une première électrode selon la revendication 12 à titre d'anode et une seconde électrode à titre de cathode.

**15.** Procédé de dosage en solution du glucose d'un échantillon, **caractérisé en ce qu'**il comprend les étapes suivantes :

a) introduction dans ledit échantillon d'un réactif d'oxydoréduction dont la réduction conduit à un changement de couleur et d'un mutant GOx selon l'une quelconque des revendications 1 à 3 ;
b) mesure de l'intensité de la coloration de l'échantillon après réaction enzymatique ;
c) comparaison de l'intensité de coloration mesurée à l'étape b) avec l'intensité mesurée pour des solutions standards ayant une teneur en glucose connue ;
d) détermination de la concentration en glucose dudit échantillon.

**16.** Procédé de dosage du glucose d'un échantillon, **caractérisé en ce qu'**il comprend les étapes suivantes :

a) introduction dans ledit échantillon d'une électrode à glucose selon la revendication 12;
b) mesure de l'intensité du courant dans l'échantillon ;
c) comparaison de l'intensité du courant mesurée à l'étape b) avec l'intensité mesurée pour des solutions standards ayant une teneur en glucose connue ;
d) détermination de la concentration en glucose dudit échantillon.

**Patentansprüche**

**1.** Mutant der Glucoseoxydase (GOx), aufweisend einen Identitätsprozentsatz von mindestens 95 % im Verhältnis zur Wildform von *Penicillium amagasakiense,* **dadurch gekennzeichnet, dass** seine Aminosäure an Position 564 in Bezug auf die Proteinsequenz der Wildform von GOx von *Penicillium amagasakiense* von SEQ. ID. Nr. 2 von einer Aminosäure substituiert ist, die aus der Gruppe ausgewählt ist, die von einem Serin (Mutant V564S), einem Threonin (Mutant V564T) oder einem Isoleucin (Mutant V564I) ausgewählt ist, und dass die Sensibilität des Mutanten gegenüber Sauerstoff geringer ist als die der Wildform von GOx von *Penicillium amagasakiense.*

**2.** Mutant der GOx nach Anspruch 1, **dadurch gekennzeichnet, dass** der Mutant V564S ebenfalls eine Substitution des Lysins an Position 424 durch eine Glutaminsäure (Mutant V564S+K424E), das Glutamin (Mutant V564S+K424Q), das Methionin (Mutant V564S+K424M) oder das Leucin (Mutant V564S+K424L) aufweist.

**3.** Mutant der GOx nach Anspruch 1 oder Anspruch 2, **dadurch gekennzeichnet, dass** er eine Aminosäurensequenz hat, die aus der Gruppe ausgewählt ist, die von den SEQ. ID. Nr. 4, 6, 8, 10, 22, 24 und 26 gebildet ist.

**4.** Nukleinsäuremolekül, **dadurch gekennzeichnet, dass** es für einen GOx-Mutanten nach einem der Ansprüche 1 bis 3 codiert.

**5.** Nukleinsäuremolekül nach Anspruch 5, **dadurch gekennzeichnet, dass** es durch Mutation des Nukleinsäuremoleküls mit der Sequenz SEQ. ID. Nr. 1 mit einem Oligonukleotidenpaar erhalten ist, das aus der Gruppe ausgewählt ist, die von den SEQ.-Paaren ID. Nr. 13 und 14; 15 und 16; 17 und 18; 19 und 20; 27 und 28; 29 und 30 und 31 und 32 gebildet ist.

**6.** Nukleinsäuremolekül nach Anspruch 4 oder Anspruch 5, **dadurch gekennzeichnet, dass** es eine Sequenz hat, die aus der Gruppe ausgewählt ist, die von den SEQ. ID. Nr. 3, 5, 7, 9, 21, 23 und 25 gebildet ist.

7. Expressionsvektor, **dadurch gekennzeichnet, dass** er ein Nukleinsäuremolekül nach einem der Ansprüche 4 bis 5 umfasst.

8. Wirtszelle, die ein Enzym nach einem der Ansprüche 1 bis 3 exprimiert, **dadurch gekennzeichnet, dass** sie mit einem Expressionsvektor nach Anspruch 7 transformiert ist.

9. Verwendung eines GOx-Mutanten nach einem der Ansprüche 1 bis 3 zum Messen der Glucosekonzentration in einer Probe.

10. Verwendung nach Anspruch 9, **dadurch gekennzeichnet, dass** die Probe biologisch ist, insbesondere Blut.

11. Glucosedosierkit, **dadurch gekennzeichnet, dass** es einen GOx-Mutanten nach einem der Ansprüche 1 bis 3 umfasst.

12. Glucoseelektrode, **dadurch gekennzeichnet, dass** sie ein leitendes Material umfasst, das mit einer Ablagerung bedeckt ist, die mindestens einen GOx-Mutanten nach einem der Ansprüche 1 bis 3 umfasst.

13. Glucosesensor, **dadurch gekennzeichnet, dass** er von einer Elektrode nach Anspruch 12 gebildet ist.

14. Glucosebiozelle, **dadurch gekennzeichnet, dass** sie eine erste Elektrode nach Anspruch 12 als Anode und eine zweite Elektrode als Kathode umfasst.

15. Glucoselösungdosierungsverfahren einer Probe, **dadurch gekennzeichnet, dass** es die folgenden Schritte umfasst:

    a) Einführen in die Probe eines Oxireduktionsreagenz, dessen Reduktion zu einer Farbveränderung und einem GOx-Mutanten nach einem der Ansprüche 1 bis 3 führt,
    b) Messen der Intensität der Färbung der Probe nach enzymatischer Reaktion,
    c) Vergleichen der in Schritt b) gemessenen Colorationsintensität mit der für Standardlösungen mit einem bekannten Glucosegehalt gemessenen Intensität,
    d) Bestimmen der Glucosekonzentration der Probe.

16. Glucoselösungdosierungsverfahren einer Probe, **dadurch gekennzeichnet, dass** es die folgenden Schritte umfasst:

    a) Einführen einer Glucoseelektrode nach Anspruch 12 in die Probe,
    b) Messen der Stromstärke in der Probe,
    c) Vergleichen der in Schritt b) gemessenen Stromstärke mit der für Standardlösungen mit einem bekannten Glucosegehalt gemessenen Stromstärke,
    d) Bestimmen der Glucosekonzentration der Probe.

**Claims**

1. A mutant of Glucose Oxidase (GOx) having at least 95 % identity with the wild-type GOx of *Penicillium amagasakiense,* **characterized in that** its amino acid at position 564, with reference to the protein sequence of the wild-type GOx of *Penicillium amagasakiense* of SEQ. ID. N°2, is substituted by an amino acid selected from the group formed by a serine (so-called V564S mutant), threonine (so-called V5644T mutant) or isoleucine (so-called V564I mutant), and **in that** said mutant is less oxygen-sensitive than the wild-type GOx of *Penicillium amagasakiense.*

2. The GOx mutant according to claim 1, **characterized in that** the V564S mutant also comprises substitution of lysine at position 424 by a glutamic acid (so-called V564S+K424E mutant), glutamine (so-called V564S+K424Q mutant), methionine (so-called V564S+K424M mutant) or leucine (so-called V564S+K424L mutant).

3. The GOx mutant according to claim 1 of claim 2 **characterized in that** it has an amino acid sequence selected from the group formed by SEQ.ID. N°s 4, 6, 8, 10, 22, 24 and 26.

4. A nucleic acid molecule **characterized in that** it encodes a GOx mutant according to any of claims 1 to 3.

**5.** The nucleic acid molecule according to claim 5, **characterized in that** it is obtained by mutation of the nucleic acid molecule of sequence SEQ.ID. N°1 with a pair of oligonucleotides selected from the group formed by the pairs of SEQ.ID. N° 13 and 14; 15 and 16; 17 and 18; 19 and 20; 27 7 and 28; 29 and 30; and 31 and 32.

**6.** The nucleic acid molecule according to claim 4 or claim 5, **characterized in that** it has a sequence selected from the group formed by SEQ.ID.N°3, 5, 7, 9, 21, 23 and 25.

**7.** An expression vector **characterized in that** it comprises a nucleic acid molecule according to any of claims 4 to 5.

**8.** A host cell expressing an enzyme according to any of claims 1 to 3, **characterized in that** it is transformed with an expression vector according to claim 7.

**9.** The use of a GOx mutant according to any of claims 1 to 3 to measure the glucose concentration of a sample.

**10.** The use according to claim 9, **characterized in that** the sample is biological, in particular it is blood.

**11.** A glucose assay kit **characterized in that** it comprises a GOx mutant according to any of claims 1 to 3.

**12.** A glucose electrode **characterized in that** it comprises a conductive material coated with a deposit comprising at least one GOx mutant according to any of claims 1 to 3.

**13.** A glucose sensor, **characterized in that** it is formed of an electrode according to claim 12.

**14.** A glucose bio-cell **characterized in that** it comprises a first electrode according to claim 12 as anode and a second electrode as cathode.

**15.** A method for solution assay of glucose in a sample, **characterized in that** it comprises the following steps:

a) adding to said sample a redox reagent, the reduction of which leads to a change in colour, and a GOx mutant according to any of claims 1 to 3;
b) measuring the colouring intensity of the sample after enzymatic reaction;
c) comparing the colouring intensity measured at step b) with the intensity measured for standard solutions having a known glucose content;
d) determining the glucose concentration of said sample.

**16.** A method for glucose assay of a sample, **characterized in that** it comprises the following steps:

a) inserting in said sample a glucose electrode according to claim 12;
b) measuring the current intensity in the sample;
c) comparing the current intensity measured at step b) with the intensity measured for standard solutions having a known glucose content;
d) determining the glucose concentration of said sample.

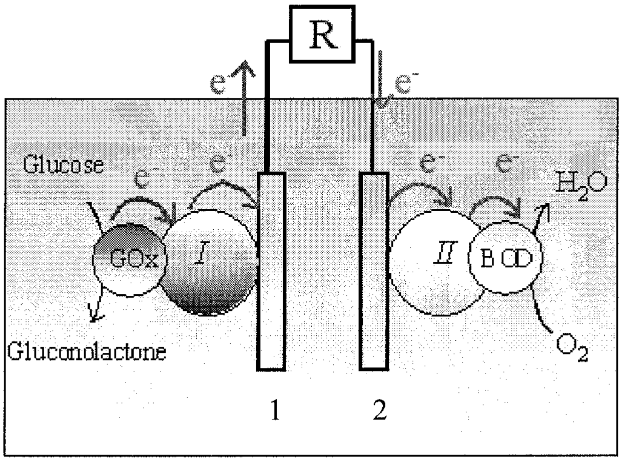


Figure 1

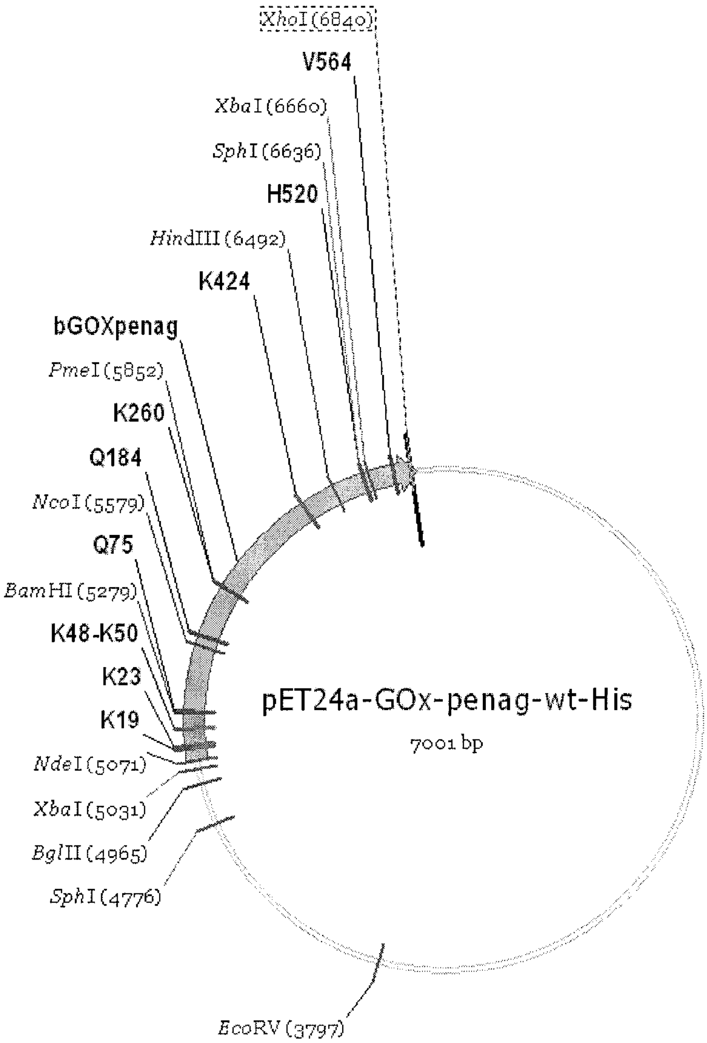


Figure 2

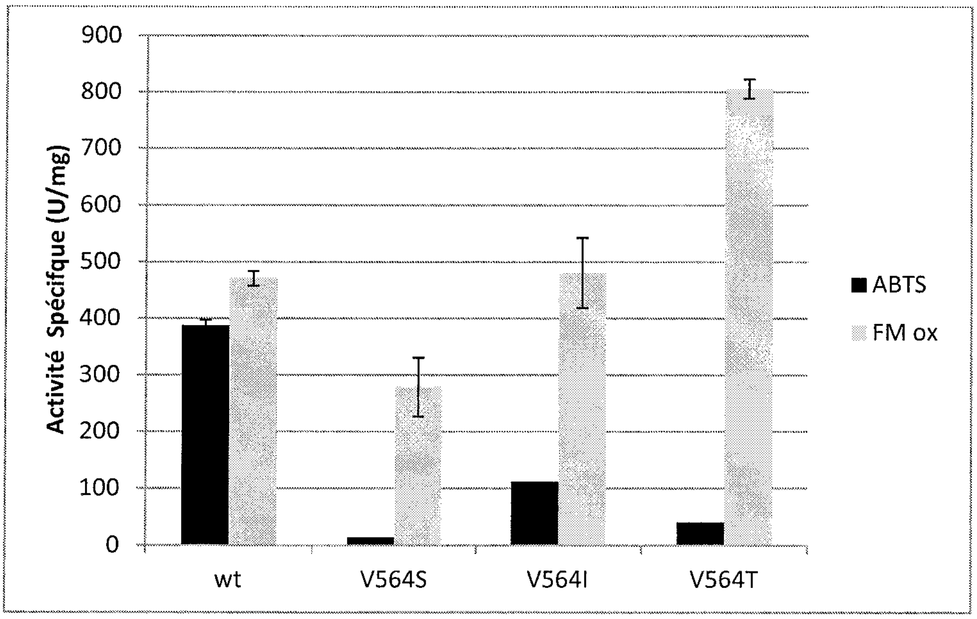


Figure 3

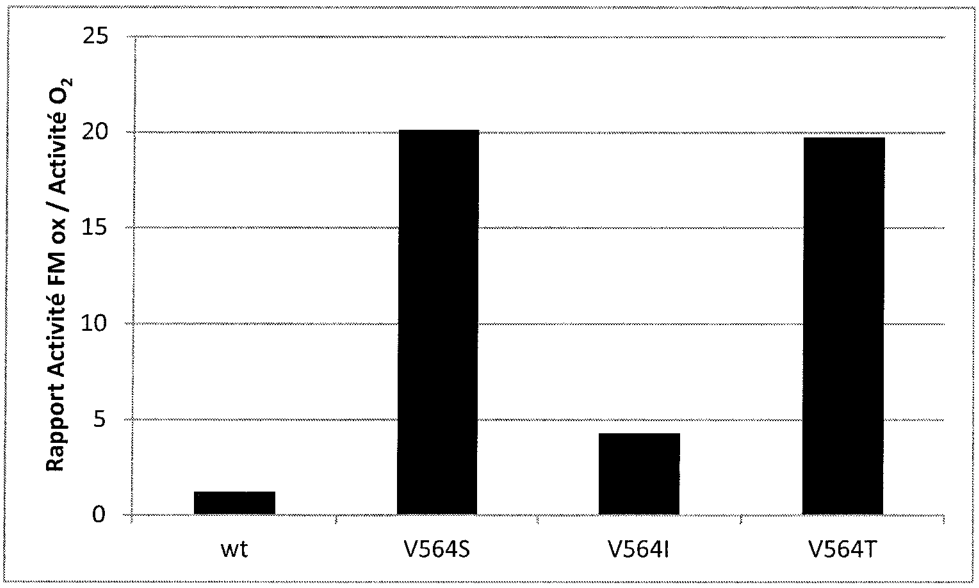


Figure 4

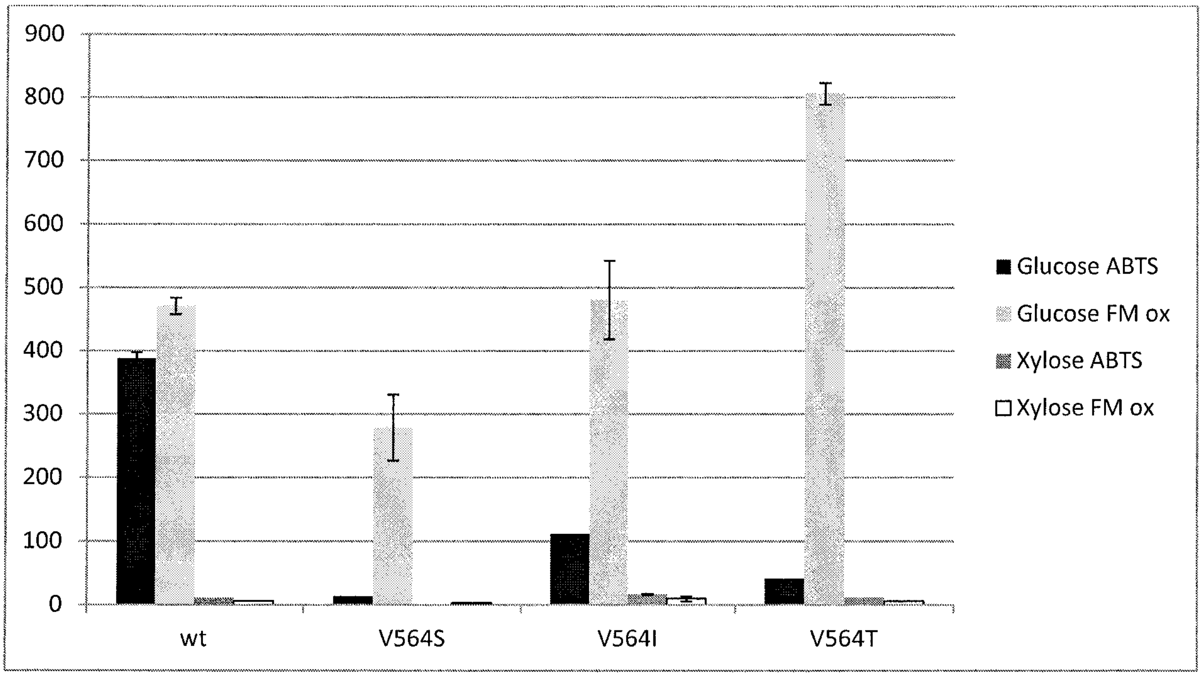


Figure 5

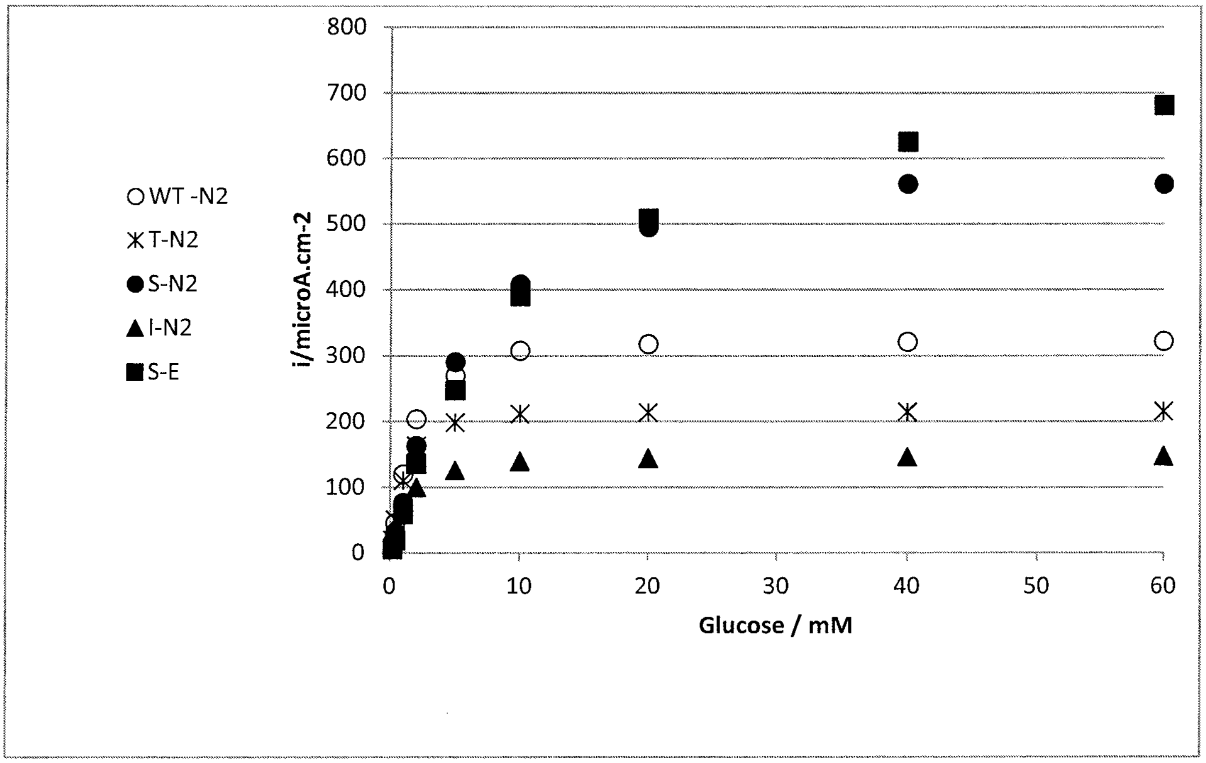


Figure 6

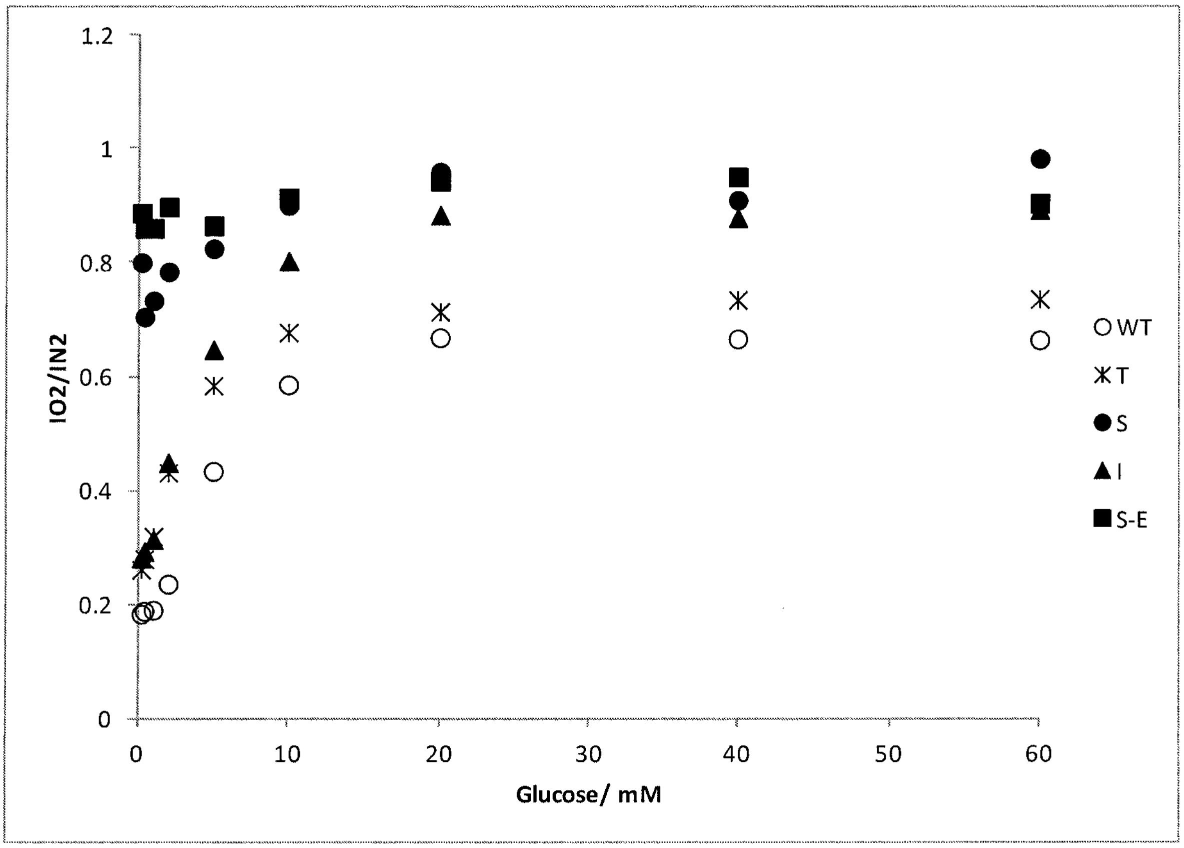


Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **WOHLFAHART et al.** *Acta. Cryst,* 1999, vol. D55, 969-977
- *Applied and Environmental Microbiology,* 1998, vol. 64 (4), 1405-1411
- **ALTSCHUL et al.** *NAR,* vol. 25, 3389-3402
- **IGARASHI et al.** *Archives of Biochemistry and Biophysocs,* 2004, vol. 428, 52-63
- **GAO et al.** *Chem. Int. ED.,* 2002, vol. 41 (5), 810-813

SEQUENCE LISTING

<110> Centre National de la Recherche Scientifique

<120> Mutants de ka glucose oxydase de Pénicillium amagasakiense

<130> CB/es - F644 333WO

<160> 32

<170> PatentIn version 3.5

<210> 1
<211> 7001
<212> DNA
<213> Pénicillium amagasakiense

<400> 1

```
tggcgaatgg gacgcgccct gtagcggcgc attaagcgcg gcgggtgtgg tggttacgcg      60
cagcgtgacc gctacacttg ccagcgccct agcgcccgct cctttcgctt tcttcccttc     120
ctttctcgcc acgttcgccg gctttccccg tcaagctcta aatcgggggc tccctttagg     180
gttccgattt agtgctttac ggcacctcga ccccaaaaaa cttgattagg gtgatggttc     240
acgtagtggg ccatcgccct gatagacggt ttttcgccct ttgacgttgg agtccacgtt     300
ctttaatagt ggactcttgt tccaaactgg aacaacactc aaccctatct cggtctattc     360
ttttgattta taagggattt tgccgatttc ggcctattgg ttaaaaaatg agctgattta     420
acaaaaattt aacgcgaatt ttaacaaaat attaacgttt acaatttcag gtggcacttt     480
tcggggaaat gtgcgcggaa cccctatttg tttatttttc taaatacatt caaatatgta     540
tccgctcatg aattaattct tagaaaaact catcgagcat caaatgaaac tgcaatttat     600
tcatatcagg attatcaata ccatattttt gaaaaagccg tttctgtaat gaaggagaaa     660
actcaccgag gcagttccat aggatggcaa gatcctggta tcggtctgcg attccgactc     720
gtccaacatc aatacaacct attaatttcc cctcgtcaaa aataaggtta tcaagtgaga     780
aatcaccatg agtgacgact gaatccggtg agaatggcaa aagtttatgc atttctttcc     840
agacttgttc aacaggccag ccattacgct cgtcatcaaa atcactcgca tcaaccaaac     900
cgttattcat tcgtgattgc gcctgagcga gacgaaatac gcgatcgctg ttaaaaggac     960
aattacaaac aggaatcgaa tgcaaccggc gcaggaacac tgccagcgca tcaacaatat    1020
tttcacctga atcaggatat tcttctaata cctggaatgc tgttttcccg gggatcgcag    1080
tggtgagtaa ccatgcatca tcaggagtac ggataaaatg cttgatggtc ggaagaggca    1140
taaattccgt cagccagttt agtctgacca tctcatctgt aacatcattg gcaacgctac    1200
ctttgccatg tttcagaaac aactctggcg catcgggctt cccatacaat cgatagattg    1260
tcgcacctga ttgcccgaca ttatcgcgag cccatttata cccatataaa tcagcatcca    1320
tgttggaatt taatcgcggc ctagagcaag acgtttcccg ttgaatatgg ctcataacac    1380
```

cccttgtatt actgtttatg taagcagaca gttttattgt tcatgaccaa aatcccttaa 1440

cgtgagtttt cgttccactg agcgtcagac cccgtagaaa agatcaaagg atcttcttga 1500

gatccttttt ttctgcgcgt aatctgctgc ttgcaaacaa aaaaaccacc gctaccagcg 1560

gtggtttgtt tgccggatca agagctacca actctttttc cgaaggtaac tggcttcagc 1620

agagcgcaga taccaaatac tgtccttcta gtgtagccgt agttaggcca ccacttcaag 1680

aactctgtag caccgcctac atacctcgct ctgctaatcc tgttaccagt ggctgctgcc 1740

agtggcgata agtcgtgtct taccgggttg gactcaagac gatagttacc ggataaggcg 1800

cagcggtcgg gctgaacggg gggttcgtgc acacagccca gcttggagcg aacgacctac 1860

accgaactga gatacctaca gcgtgagcta tgagaaagcg ccacgcttcc cgaagggaga 1920

aaggcggaca ggtatccggt aagcggcagg gtcggaacag gagagcgcac gagggagctt 1980

ccagggggaa acgcctggta tctttatagt cctgtcgggt ttcgccacct ctgacttgag 2040

cgtcgatttt tgtgatgctc gtcagggggg cggagcctat ggaaaaacgc cagcaacgcg 2100

gcctttttac ggttcctggc cttttgctgg ccttttgctc acatgttctt tcctgcgtta 2160

tcccctgatt ctgtggataa ccgtattacc gcctttgagt gagctgatac cgctcgccgc 2220

agccgaacga ccgagcgcag cgagtcagtg agcgaggaag cggaagagcg cctgatgcgg 2280

tattttctcc ttacgcatct gtgcggtatt tcacaccgca tatatggtgc actctcagta 2340

caatctgctc tgatgccgca tagttaagcc agtatacact ccgctatcgc tacgtgactg 2400

ggtcatggct gcgccccgac acccgccaac acccgctgac gcgccctgac gggcttgtct 2460

gctcccggca tccgcttaca gacaagctgt gaccgtctcc gggagctgca tgtgtcagag 2520

gttttcaccg tcatcaccga aacgcgcgag gcagctgcgg taaagctcat cagcgtggtc 2580

gtgaagcgat tcacagatgt ctgcctgttc atccgcgtcc agctcgttga gtttctccag 2640

aagcgttaat gtctggcttc tgataaagcg ggccatgtta agggcggttt tttcctgttt 2700

ggtcactgat gcctccgtgt aagggggatt tctgttcatg gggtaatga taccgatgaa 2760

acgagagagg atgctcacga tacgggttac tgatgatgaa catgcccggt tactggaacg 2820

ttgtgagggt aaacaactgg cggtatggat gcggcgggac cagagaaaaa tcactcaggg 2880

tcaatgccag cgcttcgtta atacagatgt aggtgttcca cagggtagcc agcagcatcc 2940

tgcgatgcag atccggaaca taatggtgca gggcgctgac ttccgcgttt ccagacttta 3000

cgaaacacgg aaaccgaaga ccattcatgt tgttgctcag gtcgcagacg ttttgcagca 3060

gcagtcgctt cacgttcgct cgcgtatcgg tgattcattc tgctaaccag taaggcaacc 3120

ccgccagcct agccgggtcc tcaacgacag gagcacgatc atgcgcaccc gtggggccgc 3180

catgccggcg ataatggcct gcttctcgcc gaaacgtttg gtggcgggac cagtgacgaa 3240

ggcttgagcg agggcgtgca agattccgaa taccgcaagc gacaggccga tcatcgtcgc 3300
gctccagcga aagcggtcct cgccgaaaat gacccagagc gctgccggca cctgtcctac 3360
gagttgcatg ataaagaaga cagtcataag tgcggcgacg atagtcatgc cccgcgccca 3420
ccggaaggag ctgactgggt tgaaggctct caagggcatc ggtcgagatc ccggtgccta 3480
atgagtgagc taacttacat taattgcgtt gcgctcactg cccgctttcc agtcgggaaa 3540
cctgtcgtgc cagctgcatt aatgaatcgg ccaacgcgcg gggagaggcg gtttgcgtat 3600
tgggcgccag ggtggttttt cttttcacca gtgagacggg caacagctga ttgcccttca 3660
ccgcctggcc ctgagagagt tgcagcaagc ggtccacgct ggtttgcccc agcaggcgaa 3720
aatcctgttt gatggtggtt aacggcggga tataacatga gctgtcttcg gtatcgtcgt 3780
atcccactac cgagatatcc gcaccaacgc gcagcccgga ctcggtaatg gcgcgcattg 3840
cgcccagcgc catctgatcg ttggcaacca gcatcgcagt gggaacgatg ccctcattca 3900
gcatttgcat ggtttgttga aaaccggaca tggcactcca gtcgccttcc cgttccgcta 3960
tcggctgaat ttgattgcga gtgagatatt tatgccagcc agccagacgc agacgcgccg 4020
agacagaact taatgggccc gctaacagcg cgatttgctg gtgacccaat gcgaccagat 4080
gctccacgcc cagtcgcgta ccgtcttcat gggagaaaat aatactgttg atgggtgtct 4140
ggtcagagac atcaagaaat aacgccggaa cattagtgca ggcagcttcc acagcaatgg 4200
catcctggtc atccagcgga tagttaatga tcagcccact gacgcgttgc gcgagaagat 4260
tgtgcaccgc cgctttacag gcttcgacgc cgcttcgttc taccatcgac accaccacgc 4320
tggcacccag ttgatcggcg cgagatttaa tcgccgcgac aatttgcgac ggcgcgtgca 4380
gggccagact ggaggtggca acgccaatca gcaacgactg tttgcccgcc agttgttgtg 4440
ccacgcggtt gggaatgtaa ttcagctccg ccatcgccgc ttccactttt tcccgcgttt 4500
tcgcagaaac gtggctggcc tggttcacca cgcgggaaac ggtctgataa gagacaccgg 4560
catactctgc gacatcgtat aacgttactg gtttcacatt caccaccctg aattgactct 4620
cttccgggcg ctatcatgcc ataccgcgaa aggttttgcg ccattcgatg gtgtccggga 4680
tctcgacgct ctcccttatg cgactcctgc attaggaagc agcccagtag taggttgagg 4740
ccgttgagca ccgccgccgc aaggaatggt gcatgcaagg agatggcgcc caacagtccc 4800
ccggccacgg ggcctgccac catacccacg ccgaaacaag cgctcatgag cccgaagtgg 4860
cgagcccgat cttccccatc ggtgatgtcg gcgatatagg cgccagcaac cgcacctgtg 4920
gcgccggtga tgccggccac gatgcgtccg gcgtagagga tcgagatctc gatcccgcga 4980
aattaatacg actcactata ggggaattgt gagcggataa caattcccct ctagaaataa 5040
ttttgtttaa ctttaagaag gagatataca tatgtacctg cctgcccaac agattgatgt 5100
ccagtctagt cttctcagtg accctagcaa ggttgcagga aagacctatg attacatcat 5160

tgctggtggt ggtttgactg gccttactgt tgctgccaaa ttgacagaaa accccaagat 5220

caaagtcctg gtcattgaaa agggcttcta tgagtccaac gatggagcca tcatcgagga 5280

tccaaatgct tatggacaaa tctttggcac cactgttgac cagaactacc tcaccgttcc 5340

cctgatcaac aaccgcacga acaatatcaa ggccggtaaa ggtcttggag gatcaacctt 5400

gataaacggt gactcctgga ctcgcccaga caaagtccag attgattctt gggagaaggt 5460

ctttggcatg gaaggttgga attgggacaa catgttcgag tacatgaaga aggccgaggc 5520

tgcacgtacc cctactgctg ctcagcttgc tgctggccac tccttcaatg ctacctgcca 5580

tggaaccaac ggtactgttc aatccggagc ccgtgacaac ggccagcctt ggtctcctat 5640

tatgaaggcc cttatgaaca ccgtctcggc ccttggtgtc cccgtacagc aagactttct 5700

ctgtggtcat ccacgaggtg tctctatgat catgaacaat ctcgacgaaa accaagttcg 5760

tgttgatgct gcccgtgcat ggctgcttcc caactaccag cgctcgaatt tggagatcct 5820

tactggtcag atggttggaa aggttctgtt taaacagacc gcatccggtc cccaggctgt 5880

tggtgtgaac ttcggtacta ataaggccgt caactttgac gtctttgcta agcatgaggt 5940

ccttttggct gctggctcag ctatctctcc gctgatcttg gaatattctg gcataggctt 6000

gaagtctgtt cttgatcaag ccaatgtcac tcagcttctt gatcttcctg ttggtatcaa 6060

tatgcaagat cagaccacaa ccactgtcag ttcccgtgct agttccgctg gtgctggtca 6120

gggtcaggcc gtcttcttcg ccaatttcac tgagaccttc ggtgactacg ccccccaggc 6180

cagggactta ctcaacacca agctcgacca atgggccgag gagaccgttg cgcgcggtgg 6240

tttccataat gtaactgctc tcaaagtaca atacgaaaac tatcgtaact ggctccttga 6300

cgaagacgtc gccttcgccg agcttttcat ggacaccgag ggcaagatca acttcgattt 6360

atgggatctc atccctttca ctcgtggttc cgtccatatc ctcagtagcg atccttacct 6420

atggcaattc gccaacgacc ccaaattctt cctgaacgag tttgacctcc ttggtcaagc 6480

tgccgcttcc aagcttgctc gtgatctcac tagccaaggc gctatgaagg agtacttcgc 6540

cggggagact cttccaggat acaacttggt ccagaatgct actctttccc agtggtcgga 6600

ttatgtctta cagaacttcc gtcccaactg gcatgctgtg agcagctgct ctatgatgtc 6660

tagagagctt ggtggtgtcg ttgatgctac tgccaaggtg tacggtaccc aaggcctacg 6720

tgtcattgac gggtctattc ctccgactca ggtgtcttcc catgtcatga ccattttcta 6780

cggaatggct ttgaaggttg ctgatgccat tttggatgac tatgccaaaa gtgcctcgct 6840

cgagcaccac caccaccacc actgagatcc ggctgctaac aaagcccgaa aggaagctga 6900

gttggctgct gccaccgctg agcaataact agcataaccc cttggggcct ctaaacgggt 6960

cttgaggggt tttttgctga aaggaggaac tatatccgga t 7001

<210> 2
<211> 587
<212> PRT
<213> Pénicillium amagasakiense

<400> 2

```
Tyr Leu Pro Ala Gln Gln Ile Asp Val Gln Ser Ser Leu Leu Ser Asp
1               5                   10                  15

Pro Ser Lys Val Ala Gly Lys Thr Tyr Asp Tyr Ile Ile Ala Gly Gly
            20                  25                  30

Gly Leu Thr Gly Leu Thr Val Ala Ala Lys Leu Thr Glu Asn Pro Lys
        35                  40                  45

Ile Lys Val Leu Val Ile Glu Lys Gly Phe Tyr Glu Ser Asn Asp Gly
    50                  55                  60

Ala Ile Ile Glu Asp Pro Asn Ala Tyr Gly Gln Ile Phe Gly Thr Thr
65                  70                  75                  80

Val Asp Gln Asn Tyr Leu Thr Val Pro Leu Ile Asn Asn Pro Thr Asn
                85                  90                  95

Asn Ile Lys Ala Gly Lys Gly Leu Gly Gly Ser Thr Leu Ile Asn Gly
            100                 105                 110

Asp Ser Trp Thr Arg Pro Asp Lys Val Gln Ile Asp Ser Trp Glu Lys
        115                 120                 125

Val Phe Gly Met Glu Gly Trp Asn Trp Asp Asn Met Phe Glu Tyr Met
    130                 135                 140

Lys Lys Ala Glu Ala Ala Arg Thr Pro Thr Ala Ala Gln Leu Ala Ala
145                 150                 155                 160

Gly His Ser Phe Asn Pro Thr Cys His Gly Thr Asn Pro Thr Val Gln
                165                 170                 175

Ser Gly Ala Arg Asp Asn Gly Gln Pro Trp Ser Pro Ile Met Lys Ala
            180                 185                 190

Leu Met Asn Thr Val Ser Ala Leu Gly Val Pro Val Gln Gln Asp Phe
        195                 200                 205

Leu Cys Gly His Pro Arg Gly Val Ser Met Ile Met Asn Asn Leu Asp
    210                 215                 220
```

```
Glu Asn Gln Val Arg Val Asp Ala Ala Arg Ala Trp Leu Leu Pro Asn
225                 230                 235                 240

Tyr Gln Arg Ser Asn Leu Glu Ile Leu Thr Gly Gln Met Val Gly Lys
                245                 250                 255

Val Leu Phe Lys Gln Thr Ala Ser Gly Pro Gln Ala Val Gly Val Asn
            260                 265                 270

Phe Gly Thr Asn Lys Ala Val Asn Phe Asp Val Phe Ala Lys His Glu
        275                 280                 285

Val Leu Leu Ala Ala Gly Ser Ala Ile Ser Pro Leu Ile Leu Glu Tyr
    290                 295                 300

Ser Gly Ile Gly Leu Lys Ser Val Leu Asp Gln Ala Asn Pro Thr Gln
305                 310                 315                 320

Leu Leu Asp Leu Pro Val Gly Ile Asn Met Gln Asp Gln Thr Thr Thr
                325                 330                 335

Thr Val Ser Ser Arg Ala Ser Ser Ala Gly Ala Gly Gln Gly Gln Ala
            340                 345                 350

Val Phe Phe Ala Asn Pro Thr Glu Thr Phe Gly Asp Tyr Ala Pro Gln
        355                 360                 365

Ala Arg Asp Leu Leu Asn Thr Lys Leu Asp Gln Trp Ala Glu Glu Thr
    370                 375                 380

Val Ala Arg Gly Gly Phe His Asn Pro Thr Ala Leu Lys Val Gln Tyr
385                 390                 395                 400

Glu Asn Tyr Arg Asn Trp Leu Leu Asp Glu Asp Val Ala Phe Ala Glu
                405                 410                 415

Leu Phe Met Asp Thr Glu Gly Lys Ile Asn Phe Asp Leu Trp Asp Leu
            420                 425                 430

Ile Pro Phe Thr Arg Gly Ser Val His Ile Leu Ser Ser Asp Pro Tyr
        435                 440                 445

Leu Trp Gln Phe Ala Asn Asp Pro Lys Phe Phe Leu Asn Glu Phe Asp
    450                 455                 460

Leu Leu Gly Gln Ala Ala Ala Ser Lys Leu Ala Arg Asp Leu Thr Ser
465                 470                 475                 480
```

```
Gln Gly Ala Met Lys Glu Tyr Phe Ala Gly Glu Thr Leu Pro Gly Tyr
                485                 490                 495

Asn Leu Val Gln Asn Pro Thr Leu Ser Gln Trp Ser Asp Tyr Val Leu
            500                 505                 510

Gln Asn Phe Arg Pro Asn Trp His Ala Val Ser Ser Cys Ser Met Met
        515                 520                 525

Ser Arg Glu Leu Gly Gly Val Val Asp Ala Thr Ala Lys Val Tyr Gly
    530                 535                 540

Thr Gln Gly Leu Arg Val Ile Asp Gly Ser Ile Pro Pro Thr Gln Val
545                 550                 555                 560

Ser Ser His Val Met Thr Ile Phe Tyr Gly Met Ala Leu Lys Val Ala
                565                 570                 575

Asp Ala Ile Leu Asp Asp Tyr Ala Lys Ser Ala
            580                 585
```

<210> 3
<211> 1770
<212> DNA
<213> Pénicillium amagasakiense

<400> 3

tatgtacctg cctgcccaac agattgatgt ccagtctagt cttctcagtg accctagcaa 60
ggttgcagga aagacctatg attacatcat tgctggtggt ggtttgactg gccttactgt 120
tgctgccaaa ttgacagaaa accccaagat caaagtcctg gtcattgaaa agggcttcta 180
tgagtccaac gatggagcca tcatcgagga tccaaatgct tatggacaaa tctttggcac 240
cactgttgac cagaactacc tcaccgttcc cctgatcaac aaccgcacga acaatatcaa 300
ggccggtaaa ggtcttggag gatcaacctt gataaacggt gactcctgga ctcgcccaga 360
caaagtccag attgattctt gggagaaggt ctttggcatg gaaggttgga attgggacaa 420
catgttcgag tacatgaaga aggccgaggc tgcacgtacc cctactgctg ctcagcttgc 480
tgctggccac tccttcaatg ctacctgcca tggaaccaac ggtactgttc aatccggagc 540
ccgtgacaac ggccagcctt ggtctcctat tatgaaggcc cttatgaaca ccgtctcggc 600
ccttggtgtc cccgtacagc aagactttct ctgtggtcat ccacgaggtg tctctatgat 660
catgaacaat ctcgacgaaa accaagttcg tgttgatgct gcccgtgcat ggctgcttcc 720
caactaccag cgctcgaatt tggagatcct tactggtcag atggttggaa aggttctgtt 780
taaacagacc gcatccggtc cccaggctgt tggtgtgaac ttcggtacta ataaggccgt 840

caactttgac gtctttgcta agcatgaggt ccttttggct gctggctcag ctatctctcc 900
gctgatcttg gaatattctg gcataggctt gaagtctgtt cttgatcaag ccaatgtcac 960
tcagcttctt gatcttcctg ttggtatcaa tatgcaagat cagaccacaa ccactgtcag 1020
ttcccgtgct agttccgctg gtgctggtca gggtcaggcc gtcttcttcg ccaatttcac 1080
tgagaccttc ggtgactacg ccccccaggc cagggactta ctcaacacca agctcgacca 1140
atgggccgag gagaccgttg cgcgcggtgg tttccataat gtaactgctc tcaaagtaca 1200
atacgaaaac tatcgtaact ggctccttga cgaagacgtc gccttcgccg agcttttcat 1260
ggacaccgag ggcaagatca acttcgattt atgggatctc atccctttca ctcgtggttc 1320
cgtccatatc ctcagtagcg atccttacct atggcaattc gccaacgacc ccaaattctt 1380
cctgaacgag tttgacctcc ttggtcaagc tgccgcttcc aagcttgctc gtgatctcac 1440
tagccaaggc gctatgaagg agtacttcgc cggggagact cttccaggat acaacttggt 1500
ccagaatgct actctttccc agtggtcgga ttatgtctta cagaacttcc gtcccaactg 1560
gcatgctgtg agcagctgct ctatgatgtc tagagagctt ggtggtgtcg ttgatgctac 1620
tgccaaggtg tacggtaccc aaggcctacg tgtcattgac gggtctattc ctccgactca 1680
ggtgtcttcc cattccatga ccattttcta cggaatggct ttgaaggttg ctgatgccat 1740
tttggatgac tatgccaaaa gtgcctcgct 1770

<210> 4
<211> 587
<212> PRT
<213> Pénicillium amagasakiense

<400> 4

```
Tyr Leu Pro Ala Gln Gln Ile Asp Val Gln Ser Ser Leu Leu Ser Asp
1               5                   10                  15

Pro Ser Lys Val Ala Gly Lys Thr Tyr Asp Tyr Ile Ile Ala Gly Gly
            20                  25                  30

Gly Leu Thr Gly Leu Thr Val Ala Ala Lys Leu Thr Glu Asn Pro Lys
        35                  40                  45

Ile Lys Val Leu Val Ile Glu Lys Gly Phe Tyr Glu Ser Asn Asp Gly
    50                  55                  60

Ala Ile Ile Glu Asp Pro Asn Ala Tyr Gly Gln Ile Phe Gly Thr Thr
65                  70                  75                  80

Val Asp Gln Asn Tyr Leu Thr Val Pro Leu Ile Asn Asn Pro Thr Asn
                85                  90                  95
```

```
Asn Ile Lys Ala Gly Lys Gly Leu Gly Gly Ser Thr Leu Ile Asn Gly
            100                 105                 110

Asp Ser Trp Thr Arg Pro Asp Lys Val Gln Ile Asp Ser Trp Glu Lys
        115                 120                 125

Val Phe Gly Met Glu Gly Trp Asn Trp Asp Asn Met Phe Glu Tyr Met
    130                 135                 140

Lys Lys Ala Glu Ala Ala Arg Thr Pro Thr Ala Ala Gln Leu Ala Ala
145                 150                 155                 160

Gly His Ser Phe Asn Pro Thr Cys His Gly Thr Asn Pro Thr Val Gln
                165                 170                 175

Ser Gly Ala Arg Asp Asn Gly Gln Pro Trp Ser Pro Ile Met Lys Ala
            180                 185                 190

Leu Met Asn Thr Val Ser Ala Leu Gly Val Pro Val Gln Gln Asp Phe
        195                 200                 205

Leu Cys Gly His Pro Arg Gly Val Ser Met Ile Met Asn Asn Leu Asp
    210                 215                 220

Glu Asn Gln Val Arg Val Asp Ala Ala Arg Ala Trp Leu Leu Pro Asn
225                 230                 235                 240

Tyr Gln Arg Ser Asn Leu Glu Ile Leu Thr Gly Gln Met Val Gly Lys
                245                 250                 255

Val Leu Phe Lys Gln Thr Ala Ser Gly Pro Gln Ala Val Gly Val Asn
            260                 265                 270

Phe Gly Thr Asn Lys Ala Val Asn Phe Asp Val Phe Ala Lys His Glu
        275                 280                 285

Val Leu Leu Ala Ala Gly Ser Ala Ile Ser Pro Leu Ile Leu Glu Tyr
    290                 295                 300

Ser Gly Ile Gly Leu Lys Ser Val Leu Asp Gln Ala Asn Pro Thr Gln
305                 310                 315                 320

Leu Leu Asp Leu Pro Val Gly Ile Asn Met Gln Asp Gln Thr Thr Thr
                325                 330                 335

Thr Val Ser Ser Arg Ala Ser Ser Ala Gly Ala Gly Gln Gly Gln Ala
```

```
            340                 345                 350

Val Phe Phe Ala Asn Pro Thr Glu Thr Phe Gly Asp Tyr Ala Pro Gln
        355                 360                 365

Ala Arg Asp Leu Leu Asn Thr Lys Leu Asp Gln Trp Ala Glu Glu Thr
    370                 375                 380

Val Ala Arg Gly Gly Phe His Asn Pro Thr Ala Leu Lys Val Gln Tyr
385                 390                 395                 400

Glu Asn Tyr Arg Asn Trp Leu Leu Asp Glu Asp Val Ala Phe Ala Glu
                405                 410                 415

Leu Phe Met Asp Thr Glu Gly Lys Ile Asn Phe Asp Leu Trp Asp Leu
            420                 425                 430

Ile Pro Phe Thr Arg Gly Ser Val His Ile Leu Ser Ser Asp Pro Tyr
        435                 440                 445

Leu Trp Gln Phe Ala Asn Asp Pro Lys Phe Phe Leu Asn Glu Phe Asp
    450                 455                 460

Leu Leu Gly Gln Ala Ala Ala Ser Lys Leu Ala Arg Asp Leu Thr Ser
465                 470                 475                 480

Gln Gly Ala Met Lys Glu Tyr Phe Ala Gly Glu Thr Leu Pro Gly Tyr
                485                 490                 495

Asn Leu Val Gln Asn Pro Thr Leu Ser Gln Trp Ser Asp Tyr Val Leu
            500                 505                 510

Gln Asn Phe Arg Pro Asn Trp His Ala Val Ser Ser Cys Ser Met Met
        515                 520                 525

Ser Arg Glu Leu Gly Gly Val Val Asp Ala Thr Ala Lys Val Tyr Gly
    530                 535                 540

Thr Gln Gly Leu Arg Val Ile Asp Gly Ser Ile Pro Pro Thr Gln Val
545                 550                 555                 560

Ser Ser His Ser Met Thr Ile Phe Tyr Gly Met Ala Leu Lys Val Ala
                565                 570                 575

Asp Ala Ile Leu Asp Asp Tyr Ala Lys Ser Ala
            580                 585
```

<210> 5
<211> 1768
<212> DNA
<213> Pénicillium amagasakiense

<400> 5

```
tatgtacctg cctgcccaac agattgatgt ccagtctagt cttctcagtg accctagcaa      60
ggttgcagga aagacctatg attacatcat tgctggtggt ggtttgactg gccttactgt     120
tgctgccaaa ttgacagaaa accccaagat caaagtcctg gtcattgaaa agggcttcta     180
tgagtccaac gatggagcca tcatcgagga tccaaatgct tatggacaaa tctttggcac     240
cactgttgac cagaactacc tcaccgttcc cctgatcaac aaccgcacga acaatatcaa     300
ggccggtaaa ggtcttggag atcaaccttg ataaacggtg actcctggac tcgcccagac     360
aaagtccaga ttgattcttg ggagaaggtc tttggcatga aggttggaat tgggacaaca     420
tgttcgagta catgaagaag gccgaggctg cacgtacccc tactgctgct cagcttgctg     480
ctggccactc cttcaatgct acctgccatg gaaccaacgg tactgttcaa tccggagccc     540
gtgacaacgg ccagccttgg tctcctatta tgaaggccct tatgaacacc gtctcggccc     600
ttggtgtccc cgtacagcaa gactttctct gtggtcatcc acgaggtgtc tctatgatca     660
tgaacaatct cgacgaaaac caagttcgtg ttgatgctgc ccgtgcatgg ctgcttccca     720
actaccagcg ctcgaatttg gagatcctta ctggtcagat ggttggaaag gttctgttta     780
aacagaccgc atccggtccc caggctgttg gtgtgaactt cggtactaat aaggccgtca     840
actttgacgt ctttgctaag catgaggtcc ttttggctgc tggctcagct atctctccgc     900
tgatcttgga atattctggc ataggcttga agtctgttct tgatcaagcc aatgtcactc     960
agcttcttga tcttcctgtt ggtatcaata tgcaagatca gaccacaacc actgtcagtt    1020
cccgtgctag ttccgctggt gctggtcagg gtcaggccgt cttcttcgcc aatttcactg    1080
agaccttcgg tgactacgcc ccccaggcca gggacttact caacaccaag ctcgaccaat    1140
gggccgagga gaccgttgcg cgcggtggtt tccataatgt aactgctctc aaagtacaat    1200
acgaaaacta tcgtaactgg ctccttgacg aagacgtcgc cttcgccgag cttttcatgg    1260
acaccgaggg caagatcaac ttcgatttat gggatctcat ccctttcact cgtggttccg    1320
tccatatcct cagtagcgat ccttacctat ggcaattcgc caacgacccc aaattcttcc    1380
tgaacgagtt tgacctcctt ggtcaagctg ccgcttccaa gcttgctcgt gatctcacta    1440
gccaaggcgc tatgaaggag tacttcgccg ggagactctt ccaggatac aacttggtcc     1500
agaatgctac tctttcccag tggtcggatt atgtcttaca gaacttccgt cccaactggc    1560
atgctgtgag cagctgctct atgatgtcta gagagcttgg tggtgtcgtt gatgctactg    1620
ccaaggtgta cggtacccaa ggcctacgtg tcattgacgg gtctattcct ccgactcagg    1680
tgtcttccca taccatgacc attttctacg gaatggcttt gaaggttgct gatgccattt    1740

tggatgacta tgccaaaagt gcctcgct                                        1768
```

<210> 6
<211> 587
<212> PRT
<213> Pénicillium amagasakiense

<400> 6

```
Tyr Leu Pro Ala Gln Gln Ile Asp Val Gln Ser Ser Leu Leu Ser Asp
1               5                   10                  15

Pro Ser Lys Val Ala Gly Lys Thr Tyr Asp Tyr Ile Ile Ala Gly Gly
            20                  25                  30

Gly Leu Thr Gly Leu Thr Val Ala Ala Lys Leu Thr Glu Asn Pro Lys
        35                  40                  45

Ile Lys Val Leu Val Ile Glu Lys Gly Phe Tyr Glu Ser Asn Asp Gly
    50                  55                  60

Ala Ile Ile Glu Asp Pro Asn Ala Tyr Gly Gln Ile Phe Gly Thr Thr
65                  70                  75                  80

Val Asp Gln Asn Tyr Leu Thr Val Pro Leu Ile Asn Asn Pro Thr Asn
                85                  90                  95

Asn Ile Lys Ala Gly Lys Gly Leu Gly Gly Ser Thr Leu Ile Asn Gly
            100                 105                 110

Asp Ser Trp Thr Arg Pro Asp Lys Val Gln Ile Asp Ser Trp Glu Lys
        115                 120                 125

Val Phe Gly Met Glu Gly Trp Asn Trp Asp Asn Met Phe Glu Tyr Met
    130                 135                 140

Lys Lys Ala Glu Ala Ala Arg Thr Pro Thr Ala Ala Gln Leu Ala Ala
145                 150                 155                 160

Gly His Ser Phe Asn Pro Thr Cys His Gly Thr Asn Pro Thr Val Gln
                165                 170                 175

Ser Gly Ala Arg Asp Asn Gly Gln Pro Trp Ser Pro Ile Met Lys Ala
            180                 185                 190

Leu Met Asn Thr Val Ser Ala Leu Gly Val Pro Val Gln Gln Asp Phe
        195                 200                 205
```

Leu Cys Gly His Pro Arg Gly Val Ser Met Ile Met Asn Asn Leu Asp
210 215 220

Glu Asn Gln Val Arg Val Asp Ala Ala Arg Ala Trp Leu Leu Pro Asn
225 230 235 240

Tyr Gln Arg Ser Asn Leu Glu Ile Leu Thr Gly Gln Met Val Gly Lys
245 250 255

Val Leu Phe Lys Gln Thr Ala Ser Gly Pro Gln Ala Val Gly Val Asn
260 265 270

Phe Gly Thr Asn Lys Ala Val Asn Phe Asp Val Phe Ala Lys His Glu
275 280 285

Val Leu Leu Ala Ala Gly Ser Ala Ile Ser Pro Leu Ile Leu Glu Tyr
290 295 300

Ser Gly Ile Gly Leu Lys Ser Val Leu Asp Gln Ala Asn Pro Thr Gln
305 310 315 320

Leu Leu Asp Leu Pro Val Gly Ile Asn Met Gln Asp Gln Thr Thr Thr
325 330 335

Thr Val Ser Ser Arg Ala Ser Ser Ala Gly Ala Gly Gln Gly Gln Ala
340 345 350

Val Phe Phe Ala Asn Pro Thr Glu Thr Phe Gly Asp Tyr Ala Pro Gln
355 360 365

Ala Arg Asp Leu Leu Asn Thr Lys Leu Asp Gln Trp Ala Glu Glu Thr
370 375 380

Val Ala Arg Gly Gly Phe His Asn Pro Thr Ala Leu Lys Val Gln Tyr
385 390 395 400

Glu Asn Tyr Arg Asn Trp Leu Leu Asp Glu Asp Val Ala Phe Ala Glu
405 410 415

Leu Phe Met Asp Thr Glu Gly Lys Ile Asn Phe Asp Leu Trp Asp Leu
420 425 430

Ile Pro Phe Thr Arg Gly Ser Val His Ile Leu Ser Ser Asp Pro Tyr
435 440 445

Leu Trp Gln Phe Ala Asn Asp Pro Lys Phe Phe Leu Asn Glu Phe Asp
450 455 460

```
Leu Leu Gly Gln Ala Ala Ala Ser Lys Leu Ala Arg Asp Leu Thr Ser
465                 470                 475                 480

Gln Gly Ala Met Lys Glu Tyr Phe Ala Gly Glu Thr Leu Pro Gly Tyr
                485                 490                 495

Asn Leu Val Gln Asn Pro Thr Leu Ser Gln Trp Ser Asp Tyr Val Leu
            500                 505                 510

Gln Asn Phe Arg Pro Asn Trp His Ala Val Ser Ser Cys Ser Met Met
        515                 520                 525

Ser Arg Glu Leu Gly Gly Val Val Asp Ala Thr Ala Lys Val Tyr Gly
    530                 535                 540

Thr Gln Gly Leu Arg Val Ile Asp Gly Ser Ile Pro Pro Thr Gln Val
545                 550                 555                 560

Ser Ser His Thr Met Thr Ile Phe Tyr Gly Met Ala Leu Lys Val Ala
                565                 570                 575

Asp Ala Ile Leu Asp Asp Tyr Ala Lys Ser Ala
            580                 585
```

<210> 7
<211> 1770
<212> DNA
<213> Pénicillium amagasakiense

<400> 7

tatgtacctg cctgcccaac agattgatgt ccagtctagt cttctcagtg accctagcaa 60

ggttgcagga aagacctatg attacatcat tgctggtggt ggtttgactg gccttactgt 120

tgctgccaaa ttgacagaaa accccaagat caaagtcctg gtcattgaaa agggcttcta 180

tgagtccaac gatggagcca tcatcgagga tccaaatgct tatggacaaa tctttggcac 240

cactgttgac cagaactacc tcaccgttcc cctgatcaac aaccgcacga acaatatcaa 300

ggccggtaaa ggtcttggag gatcaacctt gataaacggt gactcctgga ctcgcccaga 360

caaagtccag attgattctt gggagaaggt ctttggcatg gaaggttgga attgggacaa 420

catgttcgag tacatgaaga aggccgaggc tgcacgtacc cctactgctg ctcagcttgc 480

tgctggccac tccttcaatg ctacctgcca tggaaccaac ggtactgttc aatccggagc 540

ccgtgacaac ggccagcctt ggtctcctat tatgaaggcc cttatgaaca ccgtctcggc 600

ccttggtgtc cccgtacagc aagactttct ctgtggtcat ccacgaggtg tctctatgat 660

catgaacaat ctcgacgaaa accaagttcg tgttgatgct gcccgtgcat ggctgcttcc 720

caactaccag cgctcgaatt tggagatcct tactggtcag atggttggaa aggttctgtt 780

taaacagacc gcatccggtc cccaggctgt tggtgtgaac ttcggtacta ataaggccgt 840

caactttgac gtctttgcta agcatgaggt ccttttggct gctggctcag ctatctctcc 900

gctgatcttg gaatattctg gcataggctt gaagtctgtt cttgatcaag ccaatgtcac 960

tcagcttctt gatcttcctg ttggtatcaa tatgcaagat cagaccacaa ccactgtcag 1020

ttcccgtgct agttccgctg gtgctggtca gggtcaggcc gtcttcttcg ccaatttcac 1080

tgagaccttc ggtgactacg ccccccaggc cagggactta ctcaacacca agctcgacca 1140

atgggccgag gagaccgttg cgcgcggtgg tttccataat gtaactgctc tcaaagtaca 1200

atacgaaaac tatcgtaact ggctccttga cgaagacgtc gccttcgccg agcttttcat 1260

ggacaccgag ggcaagatca acttcgattt atgggatctc atccctttca ctcgtggttc 1320

cgtccatatc ctcagtagcg atccttacct atggcaattc gccaacgacc ccaaattctt 1380

cctgaacgag tttgacctcc ttggtcaagc tgccgcttcc aagcttgctc gtgatctcac 1440

tagccaaggc gctatgaagg agtacttcgc cggggagact cttccaggat acaacttggt 1500

ccagaatgct actctttccc agtggtcgga ttatgtctta cagaacttcc gtcccaactg 1560

gcatgctgtg agcagctgct ctatgatgtc tagagagctt ggtggtgtcg ttgatgctac 1620

tgccaaggtg tacggtaccc aaggcctacg tgtcattgac gggtctattc ctccgactca 1680

ggtgtcttcc catattatga ccattttcta cggaatggct ttgaaggttg ctgatgccat 1740

tttggatgac tatgccaaaa gtgcctcgct 1770

<210> 8

<211> 587
<212> PRT
<213> Pénicillium amagasakiense

<400> 8

```
Tyr Leu Pro Ala Gln Gln Ile Asp Val Gln Ser Ser Leu Leu Ser Asp
1               5                   10                  15

Pro Ser Lys Val Ala Gly Lys Thr Tyr Asp Tyr Ile Ile Ala Gly Gly
            20                  25                  30

Gly Leu Thr Gly Leu Thr Val Ala Ala Lys Leu Thr Glu Asn Pro Lys
        35                  40                  45

Ile Lys Val Leu Val Ile Glu Lys Gly Phe Tyr Glu Ser Asn Asp Gly
    50                  55                  60

Ala Ile Ile Glu Asp Pro Asn Ala Tyr Gly Gln Ile Phe Gly Thr Thr
65                  70                  75                  80
```

```
Val Asp Gln Asn Tyr Leu Thr Val Pro Leu Ile Asn Asn Pro Thr Asn
                85                  90                  95

Asn Ile Lys Ala Gly Lys Gly Leu Gly Gly Ser Thr Leu Ile Asn Gly
            100                 105                 110

Asp Ser Trp Thr Arg Pro Asp Lys Val Gln Ile Asp Ser Trp Glu Lys
        115                 120                 125

Val Phe Gly Met Glu Gly Trp Asn Trp Asp Asn Met Phe Glu Tyr Met
    130                 135                 140

Lys Lys Ala Glu Ala Ala Arg Thr Pro Thr Ala Ala Gln Leu Ala Ala
145                 150                 155                 160

Gly His Ser Phe Asn Pro Thr Cys His Gly Thr Asn Pro Thr Val Gln
                165                 170                 175

Ser Gly Ala Arg Asp Asn Gly Gln Pro Trp Ser Pro Ile Met Lys Ala
            180                 185                 190

Leu Met Asn Thr Val Ser Ala Leu Gly Val Pro Val Gln Gln Asp Phe
        195                 200                 205

Leu Cys Gly His Pro Arg Gly Val Ser Met Ile Met Asn Asn Leu Asp
    210                 215                 220

Glu Asn Gln Val Arg Val Asp Ala Ala Arg Ala Trp Leu Leu Pro Asn
225                 230                 235                 240

Tyr Gln Arg Ser Asn Leu Glu Ile Leu Thr Gly Gln Met Val Gly Lys
                245                 250                 255

Val Leu Phe Lys Gln Thr Ala Ser Gly Pro Gln Ala Val Gly Val Asn
            260                 265                 270

Phe Gly Thr Asn Lys Ala Val Asn Phe Asp Val Phe Ala Lys His Glu
        275                 280                 285

Val Leu Leu Ala Ala Gly Ser Ala Ile Ser Pro Leu Ile Leu Glu Tyr
    290                 295                 300

Ser Gly Ile Gly Leu Lys Ser Val Leu Asp Gln Ala Asn Pro Thr Gln
305                 310                 315                 320

Leu Leu Asp Leu Pro Val Gly Ile Asn Met Gln Asp Gln Thr Thr Thr
                325                 330                 335
```

```
Thr Val Ser Ser Arg Ala Ser Ser Ala Gly Ala Gly Gln Gly Gln Ala
            340                 345                 350

Val Phe Phe Ala Asn Pro Thr Glu Thr Phe Gly Asp Tyr Ala Pro Gln
        355                 360                 365

Ala Arg Asp Leu Leu Asn Thr Lys Leu Asp Gln Trp Ala Glu Glu Thr
    370                 375                 380

Val Ala Arg Gly Gly Phe His Asn Pro Thr Ala Leu Lys Val Gln Tyr
385                 390                 395                 400

Glu Asn Tyr Arg Asn Trp Leu Leu Asp Glu Asp Val Ala Phe Ala Glu
                405                 410                 415

Leu Phe Met Asp Thr Glu Gly Lys Ile Asn Phe Asp Leu Trp Asp Leu
            420                 425                 430

Ile Pro Phe Thr Arg Gly Ser Val His Ile Leu Ser Ser Asp Pro Tyr
        435                 440                 445

Leu Trp Gln Phe Ala Asn Asp Pro Lys Phe Phe Leu Asn Glu Phe Asp
    450                 455                 460

Leu Leu Gly Gln Ala Ala Ala Ser Lys Leu Ala Arg Asp Leu Thr Ser
465                 470                 475                 480

Gln Gly Ala Met Lys Glu Tyr Phe Ala Gly Glu Thr Leu Pro Gly Tyr
                485                 490                 495

Asn Leu Val Gln Asn Pro Thr Leu Ser Gln Trp Ser Asp Tyr Val Leu
            500                 505                 510

Gln Asn Phe Arg Pro Asn Trp His Ala Val Ser Ser Cys Ser Met Met
        515                 520                 525

Ser Arg Glu Leu Gly Gly Val Val Asp Ala Thr Ala Lys Val Tyr Gly
    530                 535                 540

Thr Gln Gly Leu Arg Val Ile Asp Gly Ser Ile Pro Pro Thr Gln Val
545                 550                 555                 560

Ser Ser His Ile Met Thr Ile Phe Tyr Gly Met Ala Leu Lys Val Ala
                565                 570                 575

Asp Ala Ile Leu Asp Asp Tyr Ala Lys Ser Ala
```

580 585

<210> 9
<211> 1770
<212> DNA
<213> Pénicillium amagasakiense

<400> 9

tatgtacctg cctgcccaac agattgatgt ccagtctagt cttctcagtg accctagcaa 60

ggttgcagga aagacctatg attacatcat tgctggtggt ggtttgactg gccttactgt 120

tgctgccaaa ttgacagaaa accccaagat caaagtcctg gtcattgaaa agggcttcta 180

tgagtccaac gatggagcca tcatcgagga tccaaatgct tatggacaaa tctttggcac 240

cactgttgac cagaactacc tcaccgttcc cctgatcaac aaccgcacga acaatatcaa 300

ggccggtaaa ggtcttggag gatcaacctt gataaacggt gactcctgga ctcgcccaga 360

caaagtccag attgattctt gggagaaggt ctttggcatg gaaggttgga attgggacaa 420

catgttcgag tacatgaaga aggccgaggc tgcacgtacc cctactgctg ctcagcttgc 480

tgctggccac tccttcaatg ctacctgcca tggaaccaac ggtactgttc aatccggagc 540

ccgtgacaac ggccagcctt ggtctcctat tatgaaggcc cttatgaaca ccgtctcggc 600

ccttggtgtc cccgtacagc aagactttct ctgtggtcat ccacgaggtg tctctatgat 660

catgaacaat ctcgacgaaa accaagttcg tgttgatgct gcccgtgcat ggctgcttcc 720

caactaccag cgctcgaatt tggagatcct tactggtcag atggttggaa aggttctgtt 780

taaacagacc gcatccggtc cccaggctgt tggtgtgaac ttcggtacta ataaggccgt 840

caactttgac gtctttgcta agcatgaggt ccttttggct gctggctcag ctatctctcc 900

gctgatcttg gaatattctg gcataggctt gaagtctgtt cttgatcaag ccaatgtcac 960

tcagcttctt gatcttcctg ttggtatcaa tatgcaagat cagaccacaa ccactgtcag 1020

ttcccgtgct agttccgctg gtgctggtca gggtcaggcc gtcttcttcg ccaatttcac 1080

tgagaccttc ggtgactacg ccccccaggc cagggactta ctcaacacca agctcgacca 1140

atgggccgag gagaccgttg cgcgcggtgg tttccataat gtaactgctc tcaaagtaca 1200

atacgaaaac tatcgtaact ggctccttga cgaagacgtc gccttcgccg agcttttcat 1260

ggacaccgag ggcgagatca acttcgattt atgggatctc atccctttca ctcgtggttc 1320

cgtccatatc ctcagtagcg atccttacct atggcaattc gccaacgacc ccaaattctt 1380

cctgaacgag tttgacctcc ttggtcaagc tgccgcttcc aagcttgctc gtgatctcac 1440

tagccaaggc gctatgaagg agtacttcgc cggggagact cttccaggat acaacttggt 1500

ccagaatgct actctttccc agtggtcgga ttatgtctta cagaacttcc gtcccaactg 1560

gcatgctgtg agcagctgct ctatgatgtc tagagagctt ggtggtgtcg ttgatgctac 1620

tgccaaggtg tacggtaccc aaggcctacg tgtcattgac gggtctattc ctccgactca 1680

ggtgtcttcc cattccatga ccattttcta cggaatggct ttgaaggttg ctgatgccat 1740

tttggatgac tatgccaaaa gtgcctcgct 1770

<210> 10

<211> 587
<212> PRT
<213> Pénicillium amagasakiense

<400> 10

```
Tyr Leu Pro Ala Gln Gln Ile Asp Val Gln Ser Ser Leu Leu Ser Asp
1               5                   10                  15

Pro Ser Lys Val Ala Gly Lys Thr Tyr Asp Tyr Ile Ile Ala Gly Gly
            20                  25                  30

Gly Leu Thr Gly Leu Thr Val Ala Ala Lys Leu Thr Glu Asn Pro Lys
        35                  40                  45

Ile Lys Val Leu Val Ile Glu Lys Gly Phe Tyr Glu Ser Asn Asp Gly
    50                  55                  60

Ala Ile Ile Glu Asp Pro Asn Ala Tyr Gly Gln Ile Phe Gly Thr Thr
65                  70                  75                  80

Val Asp Gln Asn Tyr Leu Thr Val Pro Leu Ile Asn Asn Pro Thr Asn
                85                  90                  95

Asn Ile Lys Ala Gly Lys Gly Leu Gly Gly Ser Thr Leu Ile Asn Gly
            100                 105                 110

Asp Ser Trp Thr Arg Pro Asp Lys Val Gln Ile Asp Ser Trp Glu Lys
        115                 120                 125

Val Phe Gly Met Glu Gly Trp Asn Trp Asp Asn Met Phe Glu Tyr Met
    130                 135                 140

Lys Lys Ala Glu Ala Ala Arg Thr Pro Thr Ala Ala Gln Leu Ala Ala
145                 150                 155                 160

Gly His Ser Phe Asn Pro Thr Cys His Gly Thr Asn Pro Thr Val Gln
                165                 170                 175

Ser Gly Ala Arg Asp Asn Gly Gln Pro Trp Ser Pro Ile Met Lys Ala
            180                 185                 190

Leu Met Asn Thr Val Ser Ala Leu Gly Val Pro Val Gln Gln Asp Phe
```

195 200 205

Leu Cys Gly His Pro Arg Gly Val Ser Met Ile Met Asn Asn Leu Asp
210 215 220

Glu Asn Gln Val Arg Val Asp Ala Ala Arg Ala Trp Leu Leu Pro Asn
225 230 235 240

Tyr Gln Arg Ser Asn Leu Glu Ile Leu Thr Gly Gln Met Val Gly Lys
245 250 255

Val Leu Phe Lys Gln Thr Ala Ser Gly Pro Gln Ala Val Gly Val Asn
260 265 270

Phe Gly Thr Asn Lys Ala Val Asn Phe Asp Val Phe Ala Lys His Glu
275 280 285

Val Leu Leu Ala Ala Gly Ser Ala Ile Ser Pro Leu Ile Leu Glu Tyr
290 295 300

Ser Gly Ile Gly Leu Lys Ser Val Leu Asp Gln Ala Asn Pro Thr Gln
305 310 315 320

Leu Leu Asp Leu Pro Val Gly Ile Asn Met Gln Asp Gln Thr Thr Thr
325 330 335

Thr Val Ser Ser Arg Ala Ser Ser Ala Gly Ala Gly Gln Gly Gln Ala
340 345 350

Val Phe Phe Ala Asn Pro Thr Glu Thr Phe Gly Asp Tyr Ala Pro Gln
355 360 365

Ala Arg Asp Leu Leu Asn Thr Lys Leu Asp Gln Trp Ala Glu Glu Thr
370 375 380

Val Ala Arg Gly Gly Phe His Asn Pro Thr Ala Leu Lys Val Gln Tyr
385 390 395 400

Glu Asn Tyr Arg Asn Trp Leu Leu Asp Glu Asp Val Ala Phe Ala Glu
405 410 415

Leu Phe Met Asp Thr Glu Gly Glu Ile Asn Phe Asp Leu Trp Asp Leu
420 425 430

Ile Pro Phe Thr Arg Gly Ser Val His Ile Leu Ser Ser Asp Pro Tyr
435 440 445

```
Leu Trp Gln Phe Ala Asn Asp Pro Lys Phe Phe Leu Asn Glu Phe Asp
    450                 455                 460

Leu Leu Gly Gln Ala Ala Ala Ser Lys Leu Ala Arg Asp Leu Thr Ser
465                 470                 475                 480

Gln Gly Ala Met Lys Glu Tyr Phe Ala Gly Glu Thr Leu Pro Gly Tyr
                485                 490                 495

Asn Leu Val Gln Asn Pro Thr Leu Ser Gln Trp Ser Asp Tyr Val Leu
            500                 505                 510

Gln Asn Phe Arg Pro Asn Trp His Ala Val Ser Ser Cys Ser Met Met
        515                 520                 525

Ser Arg Glu Leu Gly Gly Val Val Asp Ala Thr Ala Lys Val Tyr Gly
    530                 535                 540

Thr Gln Gly Leu Arg Val Ile Asp Gly Ser Ile Pro Pro Thr Gln Val
545                 550                 555                 560

Ser Ser His Ser Met Thr Ile Phe Tyr Gly Met Ala Leu Lys Val Ala
                565                 570                 575

Asp Ala Ile Leu Asp Asp Tyr Ala Lys Ser Ala
            580                 585
```

<210> 11
<211> 33
<212> DNA
<213> Pénicillium amagasakiense

<400> 11
ggtgtcttcc catgtcatga ccattttcta cgg 33

<210> 12
<211> 33
<212> DNA
<213> Penicillium amagasakiense

<400> 12
ccgtagaaaa tggtcatgac atgggaagac acc 33

<210> 13
<211> 33
<212> DNA
<213> Penicillium amagasakiense

<400> 13
ggtgtcttcc cattccatga ccattttcta cgg 33

<210> 14

<211> 33
<212> DNA
<213> Penicillium amagasakiense

<400> 14
ccgtagaaaa tggtcatgga atgggaagac acc 33

<210> 15
<211> 33
<212> DNA
<213> Penicillium amagasakiense

<400> 15
ggtgtcttcc cataccatga ccattttcta cgg 33

<210> 16
<211> 33
<212> DNA
<213> Penicillium amagasakiense

<400> 16
ccgtagaaaa tggtcatggt atgggaagac acc 33

<210> 17
<211> 33
<212> DNA
<213> Penicillium amagasakiense

<400> 17
ggtgtcttcc catattatga ccattttcta cgg 33

<210> 18
<211> 33
<212> DNA
<213> Penicillium amagasakiense

<400> 18
ccgtagaaaa tggtcataat atgggaagac acc 33

<210> 19
<211> 26
<212> DNA
<213> Penicillium amagasakiense

<400> 19
ggacaccgag ggcgagatca acttcg 26

<210> 20
<211> 26
<212> DNA
<213> Penicillium amagasakiense

<400> 20
cgaagttgat ctcgccctcg gtgtcc 26

<210> 21
<211> 1769
<212> DNA

<213> Penicillium aculeatum

<400> 21

```
atgtacctgc ctgcccaaca gattgatgtc cagtctagtc ttctcagtga ccctagcaag      60
gttgcaggaa agacctatga ttacatcatt gctggtggtg gtttgactgg ccttactgtt     120
gctgccaaat tgacagaaaa ccccaagatc aaagtcctgg tcattgaaaa gggcttctat     180
gagtccaacg atggagccat catcgaggat ccaaatgctt atggacaaat ctttggcacc     240
actgttgacc agaactacct caccgttccc ctgatcaaca accgcacgaa caatatcaag     300
gccggtaaag gtcttggagg atcaaccttg ataaacggtg actcctggac tcgcccagac     360
aaagtccaga ttgattcttg ggagaaggtc tttggcatgg aaggttggaa ttgggacaac     420
atgttcgagt acatgaagaa ggccgaggct gcacgtaccc ctactgctgc tcagcttgct     480
gctggccact ccttcaatgc tacctgccat ggaaccaacg gtactgttca atccggagcc     540
cgtgacaacg gccagccttg gtctcctatt atgaaggccc ttatgaacac cgtctcggcc     600
cttggtgtcc ccgtacagca agactttctc tgtggtcatc cacgaggtgt ctctatgatc     660
atgaacaatc tcgacgaaaa ccaagttcgt gttgatgctg cccgtgcatg gctgcttccc     720
aactaccagc gctcgaattt ggagatcctt actggtcaga tggttggaaa ggttctgttt     780
aaacagaccg catccggtcc ccaggctgtt ggtgtgaact tcggtactaa taaggccgtc     840
aactttgacg tctttgctaa gcatgaggtc cttttggctg ctggctcagc tatctctccg     900
ctgatcttgg aatattctgg cataggcttg aagtctgttc ttgatcaagc caatgtcact     960
cagcttcttg atcttcctgt tggtatcaat atgcaagatc agaccacaac cactgtcagt    1020
tcccgtgcta gttccgctgg tgctggtcag ggtcaggccg tcttcttcgc caatttcact    1080
gagaccttcg gtgactacgc cccccaggcc agggacttac tcaacaccaa gctcgaccaa    1140
tgggccgagg agaccgttgc gcgcggtggt ttccataatg taactgctct caaagtacaa    1200
tacgaaaact atcgtaactg gctccttgac gaagacgtcg ccttcgccga gcttttcatg    1260
gacaccgagg gccagatcaa cttcgattta tgggatctca tccctttcac tcgtggttcc    1320
gtccatatcc tcagtagcga tccttaccta tggcaattcg ccaacgaccc caaattcttc    1380
ctgaacgagt ttgacctcct tggtcaagct gccgcttcca agcttgctcg tgatctcact    1440
agccaaggcg ctatgaagga gtacttcgcc ggggagactc ttccaggata caacttggtc    1500
cagaatgcta ctctttccca gtggtcggat tatgtcttac agaacttccg tcccaactgg    1560
catgctgtga gcagctgctc tatgatgtct agagagcttg gtggtgtcgt tgatgctact    1620
gccaaggtgt acggtaccca aggcctacgt gtcattgacg ggtctattcc tccgactcag    1680
gtgtcttccc attccatgac cattttctac ggaatggctt tgaaggttgc tgatgccatt    1740
```

ttggatgact atgccaaaag tgcctcgct 1769

<210> 22
<211> 588
<212> PRT
<213> Penicillium amagasakiense

<400> 22

```
Tyr Leu Pro Ala Gln Gln Ile Asp Val Gln Ser Ser Leu Leu Ser Asp
1               5                   10                  15

Pro Ser Lys Val Ala Gly Lys Thr Tyr Asp Tyr Ile Ile Ala Gly Gly
            20                  25                  30

Gly Leu Thr Gly Leu Thr Val Ala Ala Lys Leu Thr Glu Asn Pro Lys
        35                  40                  45

Ile Lys Val Leu Val Ile Glu Lys Gly Phe Tyr Glu Ser Asn Asp Gly
    50                  55                  60

Ala Ile Ile Glu Asp Pro Asn Ala Tyr Gly Gln Ile Phe Gly Thr Thr
65                  70                  75                  80

Val Asp Gln Asn Tyr Leu Thr Val Pro Leu Ile Asn Asn Arg Thr Asn
                85                  90                  95

Asn Ile Lys Ala Gly Lys Gly Leu Gly Gly Ser Thr Leu Ile Asn Gly
            100                 105                 110

Asp Ser Trp Thr Arg Pro Asp Lys Val Gln Ile Asp Ser Trp Glu Lys
        115                 120                 125

Val Phe Gly Met Glu Gly Trp Asn Trp Asp Asn Met Phe Glu Tyr Met
    130                 135                 140

Lys Lys Ala Glu Ala Ala Arg Thr Pro Thr Ala Ala Gln Leu Ala Ala
145                 150                 155                 160

Gly His Ser Phe Asn Ala Thr Cys His Gly Thr Asn Gly Thr Val Gln
                165                 170                 175

Ser Gly Ala Arg Asp Asn Gly Gln Pro Trp Ser Pro Ile Met Lys Ala
            180                 185                 190

Leu Met Asn Thr Val Ser Ala Leu Gly Val Pro Val Gln Gln Asp Phe
        195                 200                 205
```

Leu Cys Gly His Pro Arg Gly Val Ser Met Ile Met Asn Asn Leu Asp
210 215 220

Glu Asn Gln Val Arg Val Asp Ala Ala Arg Ala Trp Leu Leu Pro Asn
225 230 235 240

Tyr Gln Arg Ser Asn Leu Glu Ile Leu Thr Gly Gln Met Val Gly Lys
245 250 255

Val Leu Phe Lys Gln Thr Ala Ser Gly Pro Gln Ala Val Gly Val Asn
260 265 270

Phe Gly Thr Asn Lys Ala Val Asn Phe Asp Val Phe Ala Lys His Glu
275 280 285

Val Leu Leu Ala Ala Gly Ser Ala Ile Ser Pro Leu Ile Leu Glu Tyr
290 295 300

Ser Gly Ile Gly Leu Lys Ser Val Leu Asp Gln Ala Asn Val Thr Gln
305 310 315 320

Leu Leu Asp Leu Pro Val Gly Ile Asn Met Gln Asp Gln Thr Thr Thr
325 330 335

Thr Val Ser Ser Arg Ala Ser Ser Ala Gly Ala Gly Gln Gly Gln Ala
340 345 350

Val Phe Phe Ala Asn Phe Thr Glu Thr Phe Gly Asp Tyr Ala Pro Gln
355 360 365

Ala Arg Asp Leu Leu Asn Thr Lys Leu Asp Gln Trp Ala Glu Glu Thr
370 375 380

Val Ala Arg Gly Gly Phe His Asn Val Thr Ala Leu Lys Val Gln Tyr
385 390 395 400

Glu Asn Tyr Arg Asn Trp Leu Leu Asp Glu Asp Val Ala Phe Ala Glu
405 410 415

Leu Phe Met Asp Thr Glu Gly Gln Ile Asn Phe Asp Leu Trp Asp Leu
420 425 430

Ile Pro Phe Thr Arg Gly Ser Val His Ile Leu Ser Ser Asp Pro Tyr
435 440 445

Leu Trp Gln Phe Ala Asn Asp Pro Lys Phe Phe Leu Asn Glu Phe Asp
450 455 460

```
Leu Leu Gly Gln Ala Ala Ala Ser Lys Leu Ala Arg Asp Leu Thr Ser
465                 470                 475                 480

Gln Gly Ala Met Lys Glu Tyr Phe Ala Gly Glu Thr Leu Pro Gly Tyr
                485                 490                 495

Asn Leu Val Gln Asn Ala Thr Leu Ser Gln Trp Ser Asp Tyr Val Leu
            500                 505                 510

Gln Asn Phe Arg Pro Asn Trp His Ala Val Ser Ser Cys Ser Met Met
        515                 520                 525

Ser Arg Glu Leu Gly Gly Val Val Asp Ala Thr Ala Lys Val Tyr Gly
    530                 535                 540

Thr Gln Gly Leu Arg Val Ile Asp Gly Ser Ile Pro Pro Thr Gln Val
545                 550                 555                 560

Ser Ser His Ser Met Thr Ile Phe Tyr Gly Met Ala Leu Lys Val Ala
                565                 570                 575

Asp Ala Ile Leu Asp Asp Tyr Ala Lys Ser Ala Ser
            580                 585
```

<210> 23
<211> 1769
<212> DNA
<213> Pénicillium amagasakiense

<400> 23

atgtacctgc ctgcccaaca gattgatgtc cagtctagtc ttctcagtga ccctagcaag 60

gttgcaggaa agacctatga ttacatcatt gctggtggtg gtttgactgg ccttactgtt 120

gctgccaaat tgacagaaaa ccccaagatc aaagtcctgg tcattgaaaa gggcttctat 180

gagtccaacg atggagccat catcgaggat ccaaatgctt atggacaaat ctttggcacc 240

actgttgacc agaactacct caccgttccc ctgatcaaca accgcacgaa caatatcaag 300

gccggtaaag gtcttggagg atcaaccttg ataaacggtg actcctggac tcgcccagac 360

aaagtccaga ttgattcttg ggagaaggtc tttggcatgg aaggttggaa ttgggacaac 420

atgttcgagt acatgaagaa ggccgaggct gcacgtaccc ctactgctgc tcagcttgct 480

gctggccact ccttcaatgc tacctgccat ggaaccaacg gtactgttca atccggagcc 540

cgtgacaacg gccagccttg gtctcctatt atgaaggccc ttatgaacac cgtctcggcc 600

cttggtgtcc ccgtacagca agactttctc tgtggtcatc cacgaggtgt ctctatgatc 660

atgaacaatc tcgacgaaaa ccaagttcgt gttgatgctg cccgtgcatg gctgcttccc 720

aactaccagc gctcgaattt ggagatcctt actggtcaga tggttggaaa ggttctgttt 780

aaacagaccg catccggtcc ccaggctgtt ggtgtgaact tcggtactaa taaggccgtc 840

aactttgacg tctttgctaa gcatgaggtc cttttggctg ctggctcagc tatctctccg 900

ctgatcttgg aatattctgg cataggcttg aagtctgttc ttgatcaagc caatgtcact 960

cagcttcttg atcttcctgt tggtatcaat atgcaagatc agaccacaac cactgtcagt 1020

tcccgtgcta gttccgctgg tgctggtcag ggtcaggccg tcttcttcgc caatttcact 1080

gagaccttcg gtgactacgc cccccaggcc agggacttac tcaacaccaa gctcgaccaa 1140

tgggccgagg agaccgttgc gcgcggtggt ttccataatg taactgctct caaagtacaa 1200

tacgaaaact atcgtaactg gctccttgac gaagacgtcg ccttcgccga gcttttcatg 1260

gacaccgagg gcatgatcaa cttcgattta tgggatctca tccctttcac tcgtggttcc 1320

gtccatatcc tcagtagcga tccttaccta tggcaattcg ccaacgaccc caaattcttc 1380

ctgaacgagt ttgacctcct tggtcaagct gccgcttcca agcttgctcg tgatctcact 1440

agccaaggcg ctatgaagga gtacttcgcc ggggagactc ttccaggata caacttggtc 1500

cagaatgcta ctctttccca gtggtcggat tatgtcttac agaacttccg tcccaactgg 1560

catgctgtga gcagctgctc tatgatgtct agagagcttg gtggtgtcgt tgatgctact 1620

gccaaggtgt acggtaccca aggcctacgt gtcattgacg ggtctattcc tccgactcag 1680

gtgtcttccc attccatgac cattttctac ggaatggctt tgaaggttgc tgatgccatt 1740

ttggatgact atgccaaaag tgcctcgct 1769

<210> 24

<211> 588
<212> PRT
<213> Penicillium amagasakiense

<400> 24

```
Tyr Leu Pro Ala Gln Gln Ile Asp Val Gln Ser Ser Leu Leu Ser Asp
1               5                   10                  15

Pro Ser Lys Val Ala Gly Lys Thr Tyr Asp Tyr Ile Ile Ala Gly Gly
            20                  25                  30

Gly Leu Thr Gly Leu Thr Val Ala Ala Lys Leu Thr Glu Asn Pro Lys
        35                  40                  45

Ile Lys Val Leu Val Ile Glu Lys Gly Phe Tyr Glu Ser Asn Asp Gly
    50                  55                  60

Ala Ile Ile Glu Asp Pro Asn Ala Tyr Gly Gln Ile Phe Gly Thr Thr
65                  70                  75                  80
```

```
Val Asp Gln Asn Tyr Leu Thr Val Pro Leu Ile Asn Asn Arg Thr Asn
                85                  90                  95

Asn Ile Lys Ala Gly Lys Gly Leu Gly Gly Ser Thr Leu Ile Asn Gly
            100                 105                 110

Asp Ser Trp Thr Arg Pro Asp Lys Val Gln Ile Asp Ser Trp Glu Lys
        115                 120                 125

Val Phe Gly Met Glu Gly Trp Asn Trp Asp Asn Met Phe Glu Tyr Met
    130                 135                 140

Lys Lys Ala Glu Ala Ala Arg Thr Pro Thr Ala Ala Gln Leu Ala Ala
145                 150                 155                 160

Gly His Ser Phe Asn Ala Thr Cys His Gly Thr Asn Gly Thr Val Gln
                165                 170                 175

Ser Gly Ala Arg Asp Asn Gly Gln Pro Trp Ser Pro Ile Met Lys Ala
            180                 185                 190

Leu Met Asn Thr Val Ser Ala Leu Gly Val Pro Val Gln Gln Asp Phe
        195                 200                 205

Leu Cys Gly His Pro Arg Gly Val Ser Met Ile Met Asn Asn Leu Asp
    210                 215                 220

Glu Asn Gln Val Arg Val Asp Ala Ala Arg Ala Trp Leu Leu Pro Asn
225                 230                 235                 240

Tyr Gln Arg Ser Asn Leu Glu Ile Leu Thr Gly Gln Met Val Gly Lys
                245                 250                 255

Val Leu Phe Lys Gln Thr Ala Ser Gly Pro Gln Ala Val Gly Val Asn
            260                 265                 270

Phe Gly Thr Asn Lys Ala Val Asn Phe Asp Val Phe Ala Lys His Glu
        275                 280                 285

Val Leu Leu Ala Ala Gly Ser Ala Ile Ser Pro Leu Ile Leu Glu Tyr
    290                 295                 300

Ser Gly Ile Gly Leu Lys Ser Val Leu Asp Gln Ala Asn Val Thr Gln
305                 310                 315                 320

Leu Leu Asp Leu Pro Val Gly Ile Asn Met Gln Asp Gln Thr Thr Thr
                325                 330                 335
```

```
Thr Val Ser Ser Arg Ala Ser Ser Ala Gly Ala Gly Gln Gly Gln Ala
            340                 345                 350

Val Phe Phe Ala Asn Phe Thr Glu Thr Phe Gly Asp Tyr Ala Pro Gln
        355                 360                 365

Ala Arg Asp Leu Leu Asn Thr Lys Leu Asp Gln Trp Ala Glu Glu Thr
    370                 375                 380

Val Ala Arg Gly Gly Phe His Asn Val Thr Ala Leu Lys Val Gln Tyr
385                 390                 395                 400

Glu Asn Tyr Arg Asn Trp Leu Leu Asp Glu Asp Val Ala Phe Ala Glu
                405                 410                 415

Leu Phe Met Asp Thr Glu Gly Met Ile Asn Phe Asp Leu Trp Asp Leu
            420                 425                 430

Ile Pro Phe Thr Arg Gly Ser Val His Ile Leu Ser Ser Asp Pro Tyr
        435                 440                 445

Leu Trp Gln Phe Ala Asn Asp Pro Lys Phe Phe Leu Asn Glu Phe Asp
    450                 455                 460

Leu Leu Gly Gln Ala Ala Ala Ser Lys Leu Ala Arg Asp Leu Thr Ser
465                 470                 475                 480

Gln Gly Ala Met Lys Glu Tyr Phe Ala Gly Glu Thr Leu Pro Gly Tyr
                485                 490                 495

Asn Leu Val Gln Asn Ala Thr Leu Ser Gln Trp Ser Asp Tyr Val Leu
            500                 505                 510

Gln Asn Phe Arg Pro Asn Trp His Ala Val Ser Ser Cys Ser Met Met
        515                 520                 525

Ser Arg Glu Leu Gly Gly Val Val Asp Ala Thr Ala Lys Val Tyr Gly
    530                 535                 540

Thr Gln Gly Leu Arg Val Ile Asp Gly Ser Ile Pro Pro Thr Gln Val
545                 550                 555                 560

Ser Ser His Ser Met Thr Ile Phe Tyr Gly Met Ala Leu Lys Val Ala
                565                 570                 575

Asp Ala Ile Leu Asp Asp Tyr Ala Lys Ser Ala Ser
```

580 585

<210> 25
<211> 1769
<212> DNA
<213> Penicillium amagasakiense

<400> 25

```
atgtacctgc ctgcccaaca gattgatgtc cagtctagtc ttctcagtga ccctagcaag      60
gttgcaggaa agacctatga ttacatcatt gctggtggtg gtttgactgg ccttactgtt     120
gctgccaaat tgacagaaaa ccccaagatc aaagtcctgg tcattgaaaa gggcttctat     180
gagtccaacg atggagccat catcgaggat ccaaatgctt atggacaaat ctttggcacc     240
actgttgacc agaactacct caccgttccc ctgatcaaca accgcacgaa caatatcaag     300
gccggtaaag gtcttggagg atcaaccttg ataaacggtg actcctggac tcgcccagac     360
aaagtccaga ttgattcttg ggagaaggtc tttggcatgg aaggttggaa ttgggacaac     420
atgttcgagt acatgaagaa ggccgaggct gcacgtaccc ctactgctgc tcagcttgct     480
gctggccact ccttcaatgc tacctgccat ggaaccaacg gtactgttca atccggagcc     540
cgtgacaacg gccagccttg gtctcctatt atgaaggccc ttatgaacac cgtctcggcc     600
cttggtgtcc ccgtacagca agactttctc tgtggtcatc cacgaggtgt ctctatgatc     660
atgaacaatc tcgacgaaaa ccaagttcgt gttgatgctg cccgtgcatg gctgcttccc     720
aactaccagc gctcgaattt ggagatcctt actggtcaga tggttggaaa ggttctgttt     780
aaacagaccg catccggtcc ccaggctgtt ggtgtgaact tcggtactaa taaggccgtc     840
aactttgacg tctttgctaa gcatgaggtc cttttggctg ctggctcagc tatctctccg     900
ctgatcttgg aatattctgg cataggcttg aagtctgttc ttgatcaagc caatgtcact     960
cagcttcttg atcttcctgt tggtatcaat atgcaagatc agaccacaac cactgtcagt    1020
tcccgtgcta gttccgctgg tgctggtcag ggtcaggccg tcttcttcgc caatttcact    1080
gagaccttcg gtgactacgc cccccaggcc agggacttac tcaacaccaa gctcgaccaa    1140
tgggccgagg agaccgttgc gcgcggtggt ttccataatg taactgctct caaagtacaa    1200
tacgaaaact atcgtaactg gctccttgac gaagacgtcg ccttcgccga gcttttcatg    1260
gacaccgagg gcttgatcaa cttcgattta tgggatctca tccctttcac tcgtggttcc    1320
gtccatatcc tcagtagcga tccttaccta tggcaattcg ccaacgaccc caaattcttc    1380
ctgaacgagt ttgacctcct tggtcaagct gccgcttcca agcttgctcg tgatctcact    1440
agccaaggcg ctatgaagga gtacttcgcc ggggagactc ttccaggata caacttggtc    1500
cagaatgcta ctctttccca gtggtcggat tatgtcttac agaacttccg tcccaactgg    1560
catgctgtga gcagctgctc tatgatgtct agagagcttg gtggtgtcgt tgatgctact    1620
gccaaggtgt acggtaccca aggcctacgt gtcattgacg ggtctattcc tccgactcag    1680
gtgtcttccc attccatgac cattttctac ggaatggctt tgaaggttgc tgatgccatt    1740
ttggatgact atgccaaaag tgcctcgct                                      1769
```

<210> 26

<211> 588
<212> PRT
<213> Penicillium amagasakiense

<400> 26

```
Tyr Leu Pro Ala Gln Gln Ile Asp Val Gln Ser Ser Leu Leu Ser Asp
1               5                   10                  15

Pro Ser Lys Val Ala Gly Lys Thr Tyr Asp Tyr Ile Ile Ala Gly Gly
            20                  25                  30

Gly Leu Thr Gly Leu Thr Val Ala Ala Lys Leu Thr Glu Asn Pro Lys
        35                  40                  45

Ile Lys Val Leu Val Ile Glu Lys Gly Phe Tyr Glu Ser Asn Asp Gly
    50                  55                  60

Ala Ile Ile Glu Asp Pro Asn Ala Tyr Gly Gln Ile Phe Gly Thr Thr
65                  70                  75                  80

Val Asp Gln Asn Tyr Leu Thr Val Pro Leu Ile Asn Asn Arg Thr Asn
                85                  90                  95

Asn Ile Lys Ala Gly Lys Gly Leu Gly Gly Ser Thr Leu Ile Asn Gly
            100                 105                 110

Asp Ser Trp Thr Arg Pro Asp Lys Val Gln Ile Asp Ser Trp Glu Lys
        115                 120                 125

Val Phe Gly Met Glu Gly Trp Asn Trp Asp Asn Met Phe Glu Tyr Met
    130                 135                 140

Lys Lys Ala Glu Ala Ala Arg Thr Pro Thr Ala Ala Gln Leu Ala Ala
145                 150                 155                 160

Gly His Ser Phe Asn Ala Thr Cys His Gly Thr Asn Gly Thr Val Gln
                165                 170                 175

Ser Gly Ala Arg Asp Asn Gly Gln Pro Trp Ser Pro Ile Met Lys Ala
            180                 185                 190

Leu Met Asn Thr Val Ser Ala Leu Gly Val Pro Val Gln Gln Asp Phe
```

195 200 205

Leu Cys Gly His Pro Arg Gly Val Ser Met Ile Met Asn Asn Leu Asp
210 215 220

Glu Asn Gln Val Arg Val Asp Ala Ala Arg Ala Trp Leu Leu Pro Asn
225 230 235 240

Tyr Gln Arg Ser Asn Leu Glu Ile Leu Thr Gly Gln Met Val Gly Lys
245 250 255

Val Leu Phe Lys Gln Thr Ala Ser Gly Pro Gln Ala Val Gly Val Asn
260 265 270

Phe Gly Thr Asn Lys Ala Val Asn Phe Asp Val Phe Ala Lys His Glu
275 280 285

Val Leu Leu Ala Ala Gly Ser Ala Ile Ser Pro Leu Ile Leu Glu Tyr
290 295 300

Ser Gly Ile Gly Leu Lys Ser Val Leu Asp Gln Ala Asn Val Thr Gln
305 310 315 320

Leu Leu Asp Leu Pro Val Gly Ile Asn Met Gln Asp Gln Thr Thr Thr
325 330 335

Thr Val Ser Ser Arg Ala Ser Ser Ala Gly Ala Gly Gln Gly Gln Ala
340 345 350

Val Phe Phe Ala Asn Phe Thr Glu Thr Phe Gly Asp Tyr Ala Pro Gln
355 360 365

Ala Arg Asp Leu Leu Asn Thr Lys Leu Asp Gln Trp Ala Glu Glu Thr
370 375 380

Val Ala Arg Gly Gly Phe His Asn Val Thr Ala Leu Lys Val Gln Tyr
385 390 395 400

Glu Asn Tyr Arg Asn Trp Leu Leu Asp Glu Asp Val Ala Phe Ala Glu
405 410 415

Leu Phe Met Asp Thr Glu Gly Leu Ile Asn Phe Asp Leu Trp Asp Leu
420 425 430

Ile Pro Phe Thr Arg Gly Ser Val His Ile Leu Ser Ser Asp Pro Tyr
435 440 445

```
Leu Trp Gln Phe Ala Asn Asp Pro Lys Phe Phe Leu Asn Glu Phe Asp
    450                 455                 460

Leu Leu Gly Gln Ala Ala Ala Ser Lys Leu Ala Arg Asp Leu Thr Ser
465                 470                 475                 480

Gln Gly Ala Met Lys Glu Tyr Phe Ala Gly Glu Thr Leu Pro Gly Tyr
                485                 490                 495

Asn Leu Val Gln Asn Ala Thr Leu Ser Gln Trp Ser Asp Tyr Val Leu
            500                 505                 510

Gln Asn Phe Arg Pro Asn Trp His Ala Val Ser Ser Cys Ser Met Met
        515                 520                 525

Ser Arg Glu Leu Gly Gly Val Val Asp Ala Thr Ala Lys Val Tyr Gly
    530                 535                 540

Thr Gln Gly Leu Arg Val Ile Asp Gly Ser Ile Pro Pro Thr Gln Val
545                 550                 555                 560

Ser Ser His Ser Met Thr Ile Phe Tyr Gly Met Ala Leu Lys Val Ala
                565                 570                 575

Asp Ala Ile Leu Asp Asp Tyr Ala Lys Ser Ala Ser
            580                 585
```

<210> 27
<211> 33
<212> DNA
<213> Penicillium amagasakiense

<400> 27
ggacaccgag ggccagatca acttcgattt atg 33

<210> 28
<211> 33
<212> DNA
<213> Penicillium amagasakiense

<400> 28
cataaatcga agttgatctg gccctcggtg tcc 33

<210> 29
<211> 33
<212> DNA
<213> Penicillium amagasakiense

<400> 29
ggacaccgag ggcatgatca acttcgattt atg 33

<210> 30

<211> 33
<212> DNA
<213> Penicillium amagasakiense

<400> 30
cataaatcga agttgatcat gccctcggtg tcc 33

<210> 31
<211> 33
<212> DNA
<213> Penicillium amagasakiense

<400> 31
ggacaccgag ggcttgatca acttcgattt atg 33

<210> 32
<211> 33
<212> DNA
<213> Penicillium amagasakiense

<400> 32
cataaatcga agttgatcaa gccctcggtg tcc 33